# EUROPEAN PATENT APPLICATION

(11) **EP 4 487 753 A1**
(43) Date of publication of application: **08.01.2025**
(21) Application number: 23763207.0
(22) Date of filing: 10.02.2023
(51) Int. Cl.: A61B 1/01, A61B 34/30, A61B 90/10

(54) **MEDICAL SYSTEM**

(30) Priority: 04.03.2022 JP 2022033258
(71) Applicant: Canon Kabushiki Kaisha, Tokyo 146-8501 (JP)
(72) Inventor: SATO Kei, Tokyo 146-8501 (JP); FUJIMOTO Kosuke, Tokyo 146-8501 (JP)
(74) Representative: Canon Europe Limited
(86) International application number: PCT/JP2023/004504
(87) International publication number: WO 2023/166945

(57) **Abstract**

A medical system includes: a base unit; a bendable unit that is attachable to and detachable from the base unit and that includes a linear member that is driven by the drive portion in a state in which the bendable unit is attached to the base unit and a bending portion that bends as the linear member is driven; and a controller that controls the drive portion. The bendable unit includes a first information storage that stores first information specific to the bendable unit. The first information includes historical information indicating whether the bendable unit has not been used or has been used. If the historical information indicates that the bendable unit has not been used, the controller permits operation of the drive portion, and, if the historical information indicates that the bendable unit has been used, the controller does not permit operation of the drive portion.

## Description

### Technical Field

The present invention relates to a medical system including a medical device whose bendable unit is attachable and detachable.

### Background Art

PTL 1 discloses a medical instrument including: an operated portion including a deforming portion that deforms and bends due to a wire; and an operating portion that deforms the deforming portion by operating the wire.

The operated portion and the operating portion are configured to be attachable and detachable from each other, and, when the operated portion and the operating portion are coupled by a magnet, motive power can be transmitted from the operating portion to the wire of the operated portion.

### Citation List

### Patent Literature

PTL 1: Japanese Patent Laid-Open No. 2013-248117

### Summary of Invention

Here, the state, the structure, and the characteristics of operated portions, which are replaced with each other, are not necessarily the same all the time. For example, a component in a state of having been used may be erroneously attached, or a replacement component having a structure different from those of previous ones may be attached. Moreover, replacement components having the same structure do not necessarily have the same characteristics due to factors such as manufacturing variation and the like.

In such a case, to date, a user has been bearing a heavy burden, because the user himself/herself has to determine whether a replacement component is in a usable state and whether it is possible to precisely control a new replacement component by using the same control method as before. Moreover, a user has been bearing a heavier burden if the user himself/herself has to change the setting of the operating portion in order to adapt to the structure and the characteristics of a new replacement component.

An object of the present invention is to automatically perform optimal control in accordance with the state, the structure, and the characteristics that are specific to a bendable unit that is a replacement component.

### Solution to Problem

One of inventions according to the present application is a medical system including: a base unit including a drive portion therein; a bendable unit that is attachable to and detachable from the base unit, the bendable unit including a linear member that is driven by the drive portion in a state in which the bendable unit is attached to the base unit and a bending portion that bends as the linear member is driven; and a controller that controls the drive portion. The bendable unit includes a first information storage that stores first information specific to the bendable unit. The first information includes historical information indicating whether the bendable unit has not been used or has been used. If the historical information indicates that the bendable unit has not been used, the controller permits operation of the drive portion, and, if the historical information indicates that the bendable unit has been used, the controller does not permit operation of the drive portion.

One of inventions according to the present application is a medical system including: a base unit including a drive portion therein; a bendable unit that is attachable to and detachable from the base unit, the bendable unit including a linear member that is driven by the drive portion in a state in which the bendable unit is attached to the base unit and a bending portion that bends as the linear member is driven; and a controller that controls the drive portion. The bendable unit includes a first information storage that stores first information specific to the bendable unit. The first information includes region number information indicating the number of regions in which the bending portion is independently bendable at different positions in a longitudinal direction of the linear member. The drive portion includes a first drive source for driving a first bending region and a second drive source for driving a second bending region that is closer than the first bending region to the base unit in the longitudinal direction. If the region number information indicates a first region number, the controller causes the first drive source to operate and does not cause the second drive source to operate, and, if the region number information indicates a second region number that is greater than the first region number, the controller causes the first drive source and the second drive source to operate.

One of inventions according to the present application is a medical system including: a base unit including a drive portion therein; a bendable unit that is attachable to and detachable from the base unit, the bendable unit including a linear member that is driven by the drive portion in a state in which the bendable unit is attached to the base unit and a bending portion that bends as the linear member is driven; and a controller that controls the drive portion. The bendable unit includes a first information storage that stores first information specific to the bendable unit. The first information includes length information indicating lengths of regions in which the bending portion is independently bendable at different positions in a longitudinal direction of the linear member. The drive portion includes a first drive source for driving a first bending region and a second drive source for driving a second bending region that is closer than the first bending region to the base unit in the longitudinal direction. If the length information indicates that a length of the first bending region is a first length, the controller drives the second drive source when a predetermined time has elapsed after driving the first drive source, and, if the length information indicates that the length of the first bending region is a second length that is greater than the first length, the controller drives the second drive source after a time longer than the predetermined time has elapsed after driving the first drive source.

One of inventions according to the present application is a medical system including: a base unit including a drive portion therein; a bendable unit that is attachable to and detachable from the base unit, the bendable unit including a linear member that is driven by the drive portion in a state in which the bendable unit is attached to the base unit and a bending portion that bends as the linear member is driven; and a controller that controls the drive portion. The bendable unit includes a first information storage that stores first information specific to the bendable unit. The first information includes response information about response characteristics of the linear member. The controller adjusts a driving amount of the drive portion based on the response information.

With the present invention, it is possible to automatically perform optimal control in accordance with the state, the structure, and the characteristics specific to a bendable unit that is a replacement component.

### Brief Description of Drawings

[Fig. 1] Fig. 1 is an overall view of a medical system.
[Fig. 2] Fig. 2 is a perspective view illustrating a medical device and a support base.
[Fig. 3A] Fig. 3A is a view illustrating a catheter.
[Fig. 3B] Fig. 3B is a view illustrating the catheter.
[Fig. 4A] Fig. 4A is a view illustrating a catheter unit.
[Fig. 4B] Fig. 4B is a view illustrating the catheter unit.
[Fig. 5A] Fig. 5A is a view illustrating a base unit and a wire driving portion.
[Fig. 5B] Fig. 5B is a view illustrating the base unit and the wire driving portion.
[Fig. 5C] Fig. 5C is a view illustrating the base unit and the wire driving portion.
[Fig. 6A] Fig. 6A is a view illustrating the wire driving portion, a coupling device, and a bending driving portion.
[Fig. 6B] Fig. 6B is a view illustrating the wire driving portion, the coupling device, and the bending driving portion.
[Fig. 6C] Fig. 6C is a view illustrating the wire driving portion, the coupling device, and the bending driving portion.
[Fig. 7A] Fig. 7A is a view illustrating attachment of the catheter unit.
[Fig. 7B] Fig. 7B is a view illustrating attachment of the catheter unit.
[Fig. 8A] Fig. 8A is a view illustrating coupling of the catheter unit and the base unit.
[Fig. 8B] Fig. 8B is a view illustrating coupling of the catheter unit and the base unit.
[Fig. 9] Fig. 9 is an exploded view illustrating coupling of the catheter unit and the base unit.
[Fig. 10] Fig. 10 is a view illustrating fixing of a drive wire by a coupling portion.
[Fig. 11] Fig. 11 is a view illustrating fixing of the drive wire by the coupling portion.
[Fig. 12] Fig. 12 is a view illustrating fixing of the drive wire by the coupling portion.
[Fig. 13] Fig. 13 is a view illustrating fixing of the drive wire by the coupling portion.
[Fig. 14] Fig. 14 is a view illustrating fixing of the drive wire by the coupling portion.
[Fig. 15] Fig. 15 is a schematic block diagram of a medical system according to a first embodiment.
[Fig. 16] Fig. 16 is a flowchart of the procedure of using a medical system.
[Fig. 17] Fig. 17 is a flowchart of control according to a first embodiment.
[Fig. 18] Fig. 18 is a flowchart of control according to a second embodiment.
[Fig. 19] Fig. 19 is a flowchart of control according to a third embodiment.
[Fig. 20] Fig. 20 is a view illustrating feedback control of a drive source.
[Fig. 21] Fig. 21 is a flowchart of control according to a fourth embodiment.
[Fig. 22] Fig. 22 is a schematic block diagram of a medical system according to a fifth embodiment.
[Fig. 23] Fig. 23 is a block diagram of a force sensor.
[Fig. 24] Fig. 24 is a graph illustrating the output characteristics of a force sensor.
[Fig. 25] Fig. 25 is a flowchart of control according to a sixth embodiment.
[Fig. 26] Fig. 26 is a flowchart of control according to a seventh embodiment.
[Fig. 27A] Fig. 27A is a perspective view illustrating an example of arrangement of electric contacts in the catheter unit and the base unit.
[Fig. 27B] Fig. 27B is a perspective view illustrating an example of arrangement of electric contacts in the catheter unit and the base unit.

### Description of Embodiments

Hereafter, the configuration of the present invention will be described with reference to the drawings. Note that the dimensions, the materials, the shapes, the dispositions, and the like of components described in the embodiments should be appropriately changed in accordance with the configuration of a device to which the present invention is applied and various conditions.

### [First Embodiment]

### <Medical System and Medical Device>

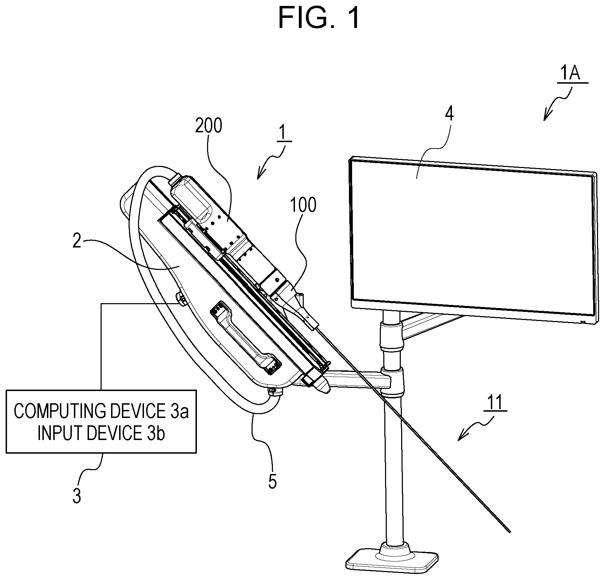

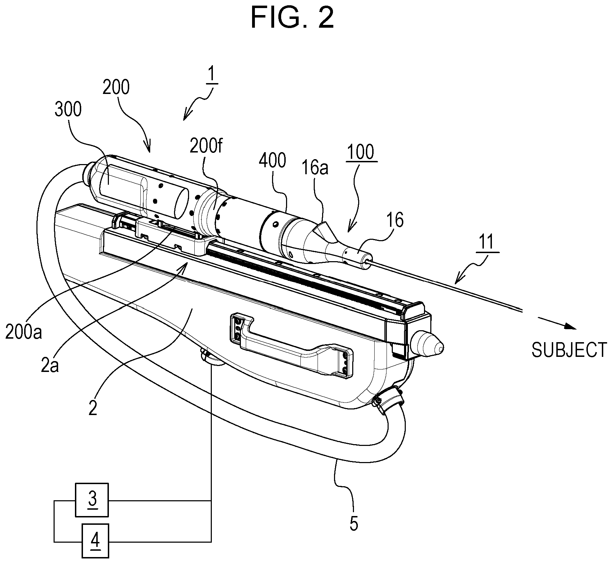

Referring to Figs. 1 and 2, a medical system 1A and a medical device 1 will be described. Fig. 1 is an overall view of the medical system 1A. Fig. 2 is a perspective view illustrating the medical device 1 and a support base 2.

The medical system 1A includes the medical device 1, the support base 2 to which the medical device 1 is attached, and a controller 3 that controls the medical device 1. In the present embodiment, the medical system 1A includes a monitor 4 as a display device.

The medical device 1 includes: a catheter unit (bending medical equipment) 100 including a catheter 11 as a bendable body; and a base unit (base portion, drive unit, attached unit) 200. The catheter unit 100 is configured to be attachable to and detachable from the base unit 200.

With the present embodiment, by inserting the catheter 11 to the inside of a subject, a user of the medical system 1A and the medical device 1 can perform works such as observation of the inside of the subject, sampling of various specimens from the inside of the subject, and treatment of the inside of the subject. As one embodiment, a user can insert the catheter 11 to the inside of a patient as a subject. For example, a user can perform a work such as observation, sampling, excision, or the like of a lung tissue by inserting the catheter 11 into a bronchus via the oral cavity of the nasal cavity of a patient.

The catheter 11 can be used as a guide (sheath) that guides a medical instrument for performing the aforementioned work. Examples of the medical instrument (tool) include an endoscope, forceps, and an ablation device. The catheter 11 itself may have the function of the medical instrument described above.

In the present embodiment, the controller 3 includes a computing device 3a and an input device 3b. The input device 3b receives commands and inputs for operating the catheter 11. The computing device 3a includes a storage that stores programs and various data for controlling the catheter, a random-access memory, and a central processing unit for executing the programs. The controller 3 may include an output portion that outputs signals for displaying images on the monitor 4.

As illustrated in Fig. 2, in the present embodiment, the medical device 1 is electrically connected to the controller 3 via a cable 5, which couples the base unit 200 of the medical device 1 and the support base 2, and the support base 2. The medical device 1 and the controller 3 may be directly connected through a cable. The medical device 1 and the controller 3 may be wirelessly connected. Although the controller 3 is illustrated as an independent component in Figs. 1 and 2, the controller 3 may be disposed inside of the base unit 200 or the support base 2.

The medical device 1 is detachably attached to the support base 2 via the base unit 200. To be more specific, an attachment portion (connection portion) 200a of the base unit 200 of the medical device 1 is detachably attached to a movement stage (receiving portion) 2a of the support base 2. Even in a state in which the attachment portion 200a of the medical device 1 is detached from the movement stage 2a, connection between the medical device 1 and the controller 3 is maintained so that the controller 3 can control the medical device 1. In the present embodiment, even in the state in which the attachment portion 200a of the medical device 1 is detached from the movement stage 2a, the medical device 1 and the support base 2 are connected through the cable 5.

A user can insert the catheter 11 to the inside of a subject by moving the medical device 1 by hand in a state in which the medical device 1 is detached from the support base 2 (a state in which the medical device 1 is detached from the movement stage 2a).

A user can use the medical device 1 in a state in which the catheter 11 is inserted into a subject and the medical device 1 is attached to the support base 2. To be specific, as the movement stage 2a moves in a state in which the medical device 1 is attached to the movement stage 2a, the medical device 1 moves. Then, an operation of moving the catheter 11 in a direction in which the catheter 11 is inserted into a subject and an operation of moving the catheter 11 in a direction in which the catheter 11 is pulled out from the subject are performed.

The medical device 1 is fixed to the movement stage 2a via the attachment portion 200a, and is moved by the movement stage 2a that is driven by a movement motor (not shown) disposed inside of the support base 2. The movement motor is controlled by the controller 3.

The medical device 1 includes a wire driving portion (linear member driving portion, line driving portion, body driving portion) 300 for driving the catheter 11. In the present embodiment, the medical device 1 is a robot catheter device that drives the catheter 11 by using the wire driving portion 300 controlled by the controller 3.

The controller 3 can control the wire driving portion 300 to perform an operation of bending the catheter 11. In the present embodiment, the wire driving portion 300 is contained in the base unit 200. To be more specific, the base unit 200 includes a base housing 200f that accommodates the wire driving portion 300. That is, the base unit 200 includes the wire driving portion 300.

Here, with respect to the extension direction of the catheter 11, an end portion where the tip of the catheter 11, which is inserted into a subject, is disposed will be referred to as a distal end. With respect to the extension direction of the catheter 11, an end portion opposite to the distal end will be referred to as a proximal end.

The catheter unit 100 includes a proximal end cover 16 that covers the proximal end side of the catheter 11. The proximal end cover 16 has a tool hole 16a. A medical instrument can be inserted into the catheter 11 via the tool hole 16a.

As described above, in the present embodiment, the catheter 11 has a function as a guiding device that guides a medical instrument to a desired position inside of a subject.

For example, in a state in which an endoscope has been inserted into the catheter 11, the catheter 11 is inserted to a target position inside of a subject. At this time, at least one of a manual operation by a user, movement of the movement stage 2a, and driving of the catheter 11 by the wire driving portion 300 is used. After the catheter 11 has reached the target position, the endoscope is pulled out from the catheter 11 via the tool hole 16a. Then, another medical instrument is inserted from the tool hole 16a, and a work such as sampling of various specimens from the inside of the subject or treatment of the inside of the subject is performed.

As described below, the catheter unit 100 is detachably attached to the base unit 200. After the medical device 1 has been used, a user can detach the catheter unit 100 from the base unit 200 and attach a new catheter unit 100 to the base unit 200 to use the medical device 1 again.

As illustrated in Fig. 2, the medical device 1 includes an operating portion 400. In the present embodiment, the operating portion 400 is included in the catheter unit 100. A user operates the operating portion 400 when fixing the catheter unit 100 to the base unit 200 and when detaching the catheter unit 100 from the base unit 200.

By connecting the monitor 4 and an endoscope inserted into the catheter 11, it is possible to cause the monitor 4 to display an image captured by the endoscope. Moreover, by connecting the monitor 4 and the controller 3, it is possible to cause the monitor 4 to display information about the state of the medical device 1 and the control of the medical device 1. For example, it is possible to cause the monitor 4 to display information about the position of the catheter 11 inside of a subject and navigation of the catheter 11 inside of the subject. The monitor 4, the controller 3, and the endoscope may be connected by wire or may be connected wirelessly. The monitor 4 and the controller 3 may be connected via the support base 2.

### <Catheter>

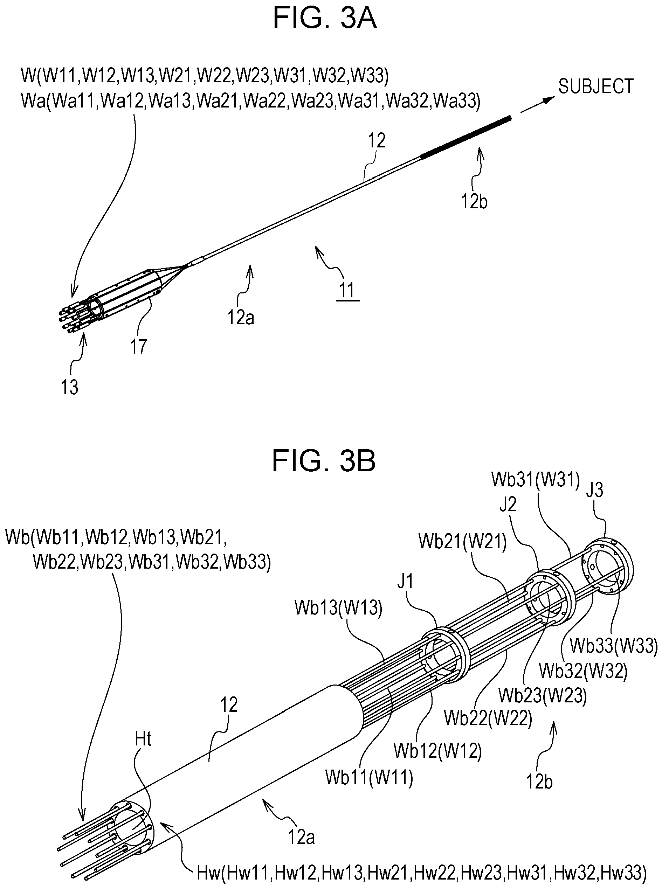

Referring to Figs. 3A and 3B, the catheter 11 as a bendable body will be described. Figs. 3A and 3B are views illustrating the catheter 11. Fig. 3A is a view illustrating the entirety of the catheter 11. Fig. 3B is an enlarged view of the catheter 11.

The catheter 11 includes a bending portion (bending body, catheter body) 12 and a bending driving portion (catheter driving portion) 13 that is configured to bend the bending portion 12. The bending driving portion 13 is configured to bend the bending portion 12 by receiving a driving force of the wire driving portion 300 via a coupling device 21 described below.

The catheter 11 extends along an insertion direction in which the catheter 11 is inserted into a subject. The extension direction (longitudinal direction) of the catheter 11 is the same as the extension direction (longitudinal direction) of the bending portion 12 and the extension direction (longitudinal direction) of first to ninth drive wires (W11 to W33) described below.

The bending driving portion 13 includes a plurality of drive wires (drive lines, linear members, linear actuators) connected to the bending portion 12. To be specific, the bending driving portion 13 includes a first drive wire W11, a second drive wire W12, a third drive wire W13, a fourth drive wire W21, a fifth drive wire W22, a sixth drive wire W23, a seventh drive wire W31, an eighth drive wire W32, and a ninth drive wire W33.

Each of the first to ninth drive wires (W11 to W33) includes a held portion (held shaft, rod) Wa. To be specific, the first drive wire W11 includes a first held portion Wall. The second drive wire W12 includes a second held portion Wa12. The third drive wire W13 includes a third held portion Wa13. The fourth drive wire W21 includes a fourth held portion Wa21. The fifth drive wire W22 includes a fifth held portion Wa22. The sixth drive wire W23 includes a sixth held portion Wa23. The seventh drive wire W31 includes a seventh held portion Wa31. The eighth drive wire W32 includes an eighth held portion Wa32. The ninth drive wire W33 includes a ninth held portion Wa33.

In the present embodiment, the first to ninth held portions (Wall to Wa33) have the same shape.

Each of the first to ninth drive wires (W11 to W33) includes a wire body (line body, linear body) Wb having flexibility. To be specific, the first drive wire W11 includes a first wire body Wb11. The second drive wire W12 includes a second wire body Wb12. The third drive wire W13 includes a third wire body Wb13. The fourth drive wire W21 includes a fourth wire body Wb21.

The fifth drive wire W22 includes a fifth wire body Wb22. The sixth drive wire W23 includes a sixth wire body Wb23. The seventh drive wire W31 includes a seventh wire body Wb31. The eighth drive wire W32 includes an eighth wire body Wb32. The ninth drive wire W33 includes a ninth wire body Wb33.

In the present embodiment, the first to third wire bodies (Wb11 to Wb13) have the same shape. The fourth to sixth wire bodies (Wb21 to Wb23) have the same shape.

The seventh to ninth wire bodies (Wb31 to Wb33) have the same shape. In the present embodiment, the first to ninth wire bodies (Wb11 to Wb33) have the same shape except for the lengths thereof.

The first to ninth held portions (Wall to Wa33) are fixed to the first to ninth wire bodies (Wb11 to Wb33) at the proximal ends of the first to ninth wire bodies (Wb11 to Wb33).

The first to ninth drive wires (W11 to W33) are inserted into the bending portion 12 via a wire guide 17 and fixed.

Any one of the first to ninth drive wires (W11 to W33) can be referred to as a drive wire W. In the present embodiment, the first to ninth drive wires (W11 to W33) have the same shape except for the lengths of the first to ninth wire bodies (Wb11 to Wb33)

In the present embodiment, the bending portion 12 is a tubular member having flexibility and having a passage Ht for inserting a medical instrument.

A wall of the bending portion 12 has a plurality of wire holes respectively for inserting the first to ninth drive wires (W11 to W33). To be specific, the wall of the bending portion 12 has a first wire hole Hw11, a second wire hole Hw12, and a third wire hole Hw13. Moreover, the wall of the bending portion 12 has a fourth wire hole Hw21, a fifth wire hole Hw22, and a sixth wire hole Hw23. Moreover, the wall of the bending portion 12 has a seventh wire hole Hw31, an eighth wire hole Hw32, and a ninth wire hole Hw33. The first to ninth wire holes Hw (Hw11 to Hw33) respectively correspond to the first to ninth drive wires (W11 to W33). The numeral after the symbol Hw represents the numeral of the corresponding drive wire. For example, the first drive wire W11 is inserted into the first wire hole Hw11.

Any one of the first to ninth wire holes (Hw11 to Hw33) can be referred to as a wire hole Hw. In the present embodiment, the first to ninth wire holes (Hw11 to Hw33) have the same shape.

The bending portion 12 includes a middle region 12a and a bending region 12b. The bending region 12b is disposed at the distal end of the bending portion 12, and a first guide ring J1, a second guide ring J2, and a third guide ring J3 are disposed in the bending region 12b. The bending region 12b is a region that allows the degree and the direction of bending of the bending portion 12 to be controlled by moving the first guide ring J1, the second guide ring J2, and the third guide ring J3 by using the bending driving portion 13. In Fig. 3B, illustration of a part of the bending portion 12 that covers the first to third guide rings (J1 to J3) is omitted.

A medical instrument is guided by the passage Ht and the first to third guide rings (J1 to J3) to the distal end of the catheter 11.

Each of the first to ninth drive wires (W11 to W33) is fixed to a corresponding one of the first to third guide rings (J1 to J3) through the middle region 12a.

To be specific, the first drive wire W11, the second drive wire W12, and the third drive wire W13 are fixed to the first guide ring J1. The fourth drive wire W21, the fifth drive wire W22, and the sixth drive wire W23 extend through the first guide ring J1, and are fixed to the second guide ring J2. The seventh drive wire W31, the eighth drive wire W32, and the ninth drive wire W33 extend through the first guide ring J1 and the second guide ring J2, and are fixed to the third guide ring J3.

The medical device 1 can bend the bending portion 12 in a direction that intersects the extension direction of the catheter 11 by driving the bending driving portion 13 by using the wire driving portion 300. To be specific, by moving each of the first to ninth drive wires (W11 to W33) in the extension direction of the bending portion 12, it is possible to bend the bending region 12b of the bending portion 12 in a direction that intersects the extension direction via the first to third guide rings (J1 to J3).

A user can insert the catheter 11 into a target portion inside of a subject by using at least one of: movement of the medical device 1 by hand or by using the movement stage 2a; and bending of the bending portion 12.

### <Catheter Unit>

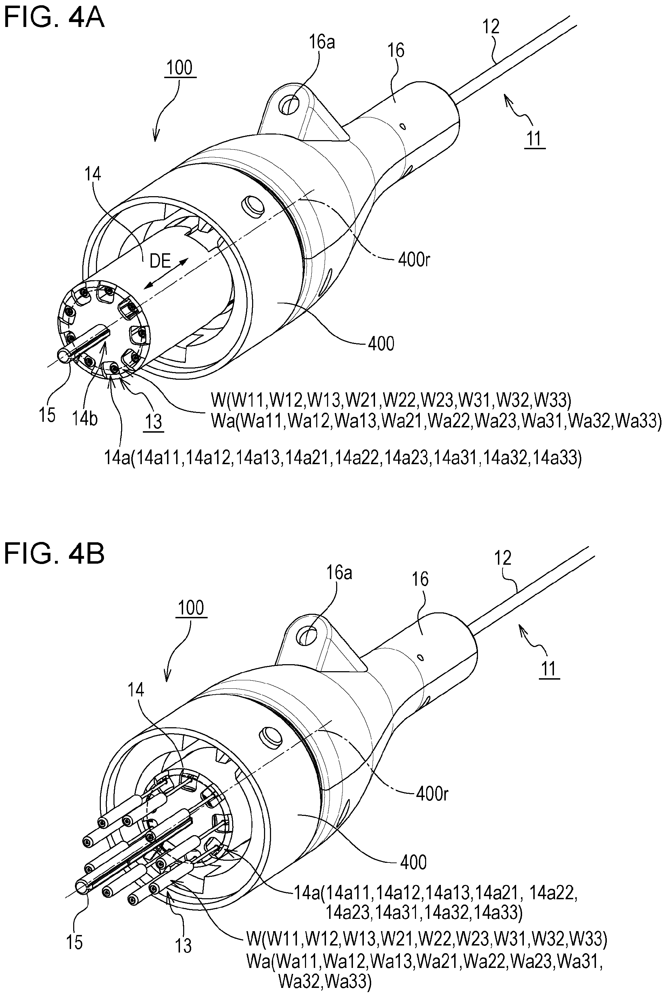

Referring to Figs. 4A and 4B, the catheter unit 100 will be described.

Figs. 4A and 4B are views illustrating the catheter unit 100. Fig. 4A is a view illustrating the catheter unit 100 in a state in which a wire cover 14 described below is at a covering position. Fig. 4B is a view illustrating the catheter unit 100 in a state in which the wire cover 14 described below is at an exposing position.

The catheter unit 100 includes the catheter 11, which includes the bending portion 12 and the bending driving portion 13, and the proximal end cover 16, which covers the proximal end of the catheter 11. The catheter unit 100 includes the cover (wire cover) 14 for covering and protecting the first to ninth drive wires (W11 to W33) as a plurality of drive wires.

The catheter unit 100 is attachable to and detachable from the base unit 200 along attachment/detachment directions DE. The attachment direction of the catheter unit 100 to the base unit 200 and the detachment direction of the catheter unit 100 from the base unit 200 are parallel to the attachment/detachment directions DE.

The proximal end cover (frame body, bending portion housing, catheter housing) 16 is a cover that covers a part of the catheter 11. The proximal end cover 16 has the tool hole 16a for inserting a medical instrument into the passage Ht of the bending portion 12.

The wire cover 14 has a plurality of exposure holes (wire cover holes, cover holes) respectively for inserting the first to ninth drive wires (W11 to W33). The wire cover 14 has a first exposure hole 14a11, a second exposure hole 14a12, a third exposure hole 14a13, a fourth exposure hole 14a21, a fifth exposure hole 14a22, a sixth exposure hole 14a23, a seventh exposure hole 14a31, an eighth exposure hole 14a32, and a ninth exposure hole 14a33. The first to ninth exposure holes (14a11 to 14a33) respectively correspond to the first to ninth drive wires (W11 to W33). The numeral after the symbol 14a represents the numeral of the corresponding drive wire. For example, the first drive wire W11 is inserted into the first exposure hole 14a11.

Any one of the first to ninth exposure holes (14a11 to 14a33) can be referred to as an exposure hole 14a. In the present embodiment, the first to ninth exposure holes (14a11 to 14a33) have the same shape.

The wire cover 14 can move to a covering position (see Fig. 4A) where the wire cover 14 covers the first to ninth drive wires (W11 to W33) and a cover retracted position (see Fig. 4B) where the wire cover 14 is retracted from the covering position. The cover retracted position can also be referred to as an exposing position where the wire cover 14 exposes the first to ninth drive wires (W11 to W33).

Before the catheter unit 100 is attached to the base unit 200, the wire cover 14 is located at the covering position. When the catheter unit 100 is attached to the base unit 200, the wire cover 14 moves from the covering position to the exposing position along the attachment/detachment directions DE.

In the present embodiment, after moving from the covering position to the exposing position, the wire cover 14 is retained at the exposing position. Accordingly, after the catheter unit 100 has been attached to the base unit 200, the wire cover 14 is retained at the exposing position even when the catheter unit 100 is detached from the base unit 200.

When the wire cover 14 is at the exposing position, the first to ninth held portions (Wall to Wa33) of the first to ninth drive wires (W11 to W33) are exposed. As a result, coupling of the bending driving portion 13 and the coupling device 21 described below is allowed. When the wire cover 14 is at the exposing position, the first to ninth held portions (Wall to Wa33) of the first to ninth drive wires (W11 to W33) protrude from the first to ninth exposure holes (14a11 to 14a33). To be more specific, the first to ninth held portions (Wall to Wa33) protrude from the first to ninth exposure holes (14a11 to 14a33) in an attachment direction Da described below.

As illustrated in Fig. 4B, the first to ninth drive wires (W11 to W33) are arranged along a circle (imaginary circle) having a predetermined radius.

In the present embodiment, the catheter unit 100 includes a key shaft (key, catheter-side key) 15. In the present embodiment, the key shaft 15 extends in the attachment/detachment directions DE. The wire cover 14 has a shaft hole 14b through which the key shaft 15 extends.

The key shaft 15 is engageable with a key receiving portion 22 described below. As the key shaft 15 engages with the key receiving portion 22, with respect to the circumferential direction of the circle (imaginary circle) along which the first to ninth drive wires (W11 to W33) are arranged, movement of the catheter unit 100 relative to the base unit 200 is limited in a predetermined range.

In the present embodiment, the catheter unit 100 includes the operating portion 400. The operating portion 400 is configured to be movable (rotatable) relative to the proximal end cover 16 and the bending driving portion 13.

The operating portion 400 is rotatable around a rotation axis 400r. The rotation axis 400r of the operating portion 400 extends in the attachment/detachment directions DE.

In a state in which the catheter unit 100 is attached to the base unit 200, the operating portion 400 is configured to be movable (rotatable) relative to the base unit 200. To be more specific, the operating portion 400 is configured to be movable (rotatable) relative to the base housing 200f, the wire driving portion 300, and the coupling device 21 described below.

### <Base Unit>

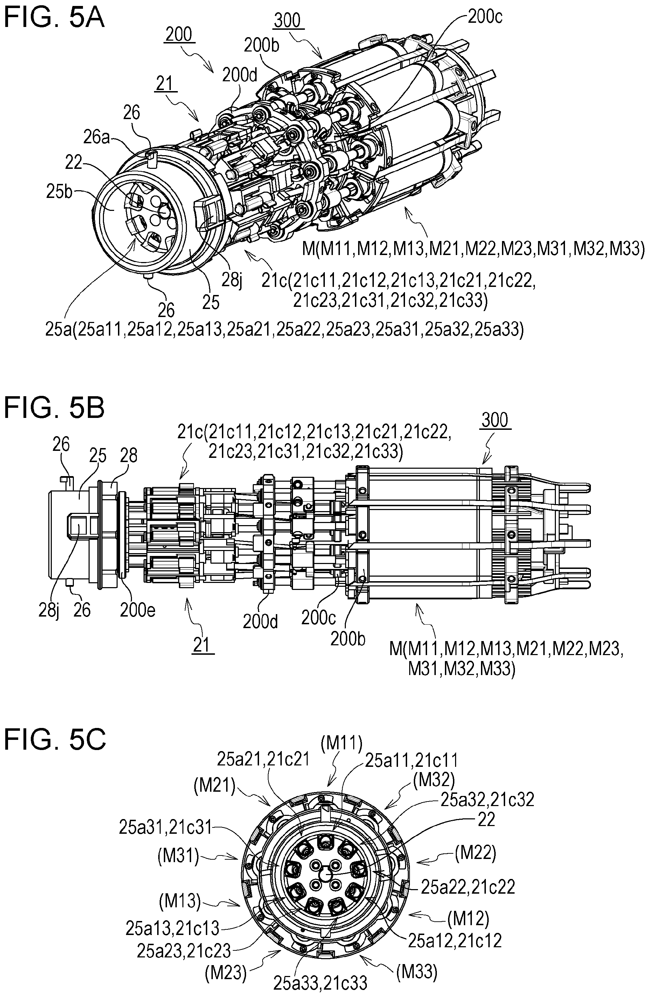

Referring to Figs. 5A to 5C, the base unit 200 and the wire driving portion 300 will be described.

Figs. 5A to 5C are views illustrating the base unit 200 and the wire driving portion 300. Fig. 5A is a perspective view illustrating the internal structure of the base unit 200. Fig. 5B is a side view illustrating the internal structure of the base unit 200. Fig. 5C is a view of the base unit 200 as seen along the attachment/detachment directions DE.

The wire driving portion 300 includes a plurality of drive sources (motors). In the present embodiment, the wire driving portion 300 includes a first drive source M11, a second drive source M12, a third drive source M13, a fourth drive source M21, a fifth drive source M22, a sixth drive source M23, a seventh drive source M31, an eighth drive source M32, and a ninth drive source M33.

Any one of the first to ninth drive sources (M11 to M33) can be referred to as a drive source M. In the present embodiment, the first to ninth drive sources (M11 to M33) have the same configuration.

The base unit 200 includes the coupling device 21. The coupling device 21 is accommodated in the base housing 200f. The coupling device 21 is connected to the wire driving portion 300. The coupling device 21 includes a plurality of coupling portions. In the present embodiment, the coupling device 21 includes a first coupling portion 21c11, a second coupling portion 21c12, a third coupling portion 21c13, a fourth coupling portion 21c21, a fifth coupling portion 21c22, a sixth coupling portion 21c23, a seventh coupling portion 21c31, an eighth coupling portion 21c32, and a ninth coupling portion 21c33.

Any one of the first to ninth coupling portions (21c11 to 21c33) can be referred to as a coupling portion 21c. In the present embodiment, the first to ninth coupling portions (21c11 to 21c33) have the same configuration.

Each of the plurality of coupling portions is connected to a corresponding one of the plurality of drive sources and is driven by the corresponding one of the plurality of drive sources. To be specific, the first coupling portion 21c11 is connected to the first drive source M11 and is driven by the first drive source M11. The second coupling portion 21c12 is connected to the second drive source M12 and is driven by the second drive source M12. The third coupling portion 21c13 is connected to the third drive source M13 and is driven by the third drive source M13. The fourth coupling portion 21c21 is connected to the fourth drive source M21 and is driven by the fourth drive source M21. The fifth coupling portion 21c22 is connected to the fifth drive source M22 and is driven by the fifth drive source M22. The sixth coupling portion 21c23 is connected to the sixth drive source M23 and is driven by the sixth drive source M23. The seventh coupling portion 21c31 is connected to the seventh drive source M31 and is driven by the seventh drive source M31. The eighth coupling portion 21c32 is connected to the eighth drive source M32 and is driven by the eighth drive source M32. The ninth coupling portion 21c33 is connected to the ninth drive source M33 and is driven by the ninth drive source M33.

As described below, the bending driving portion 13, including the first to ninth drive wires (W11 to W33), is coupled to the coupling device 21. The bending driving portion 13 bends the bending portion 12 by receiving a driving force of the wire driving portion 300 via the coupling device 21.

The drive wire W is coupled to the coupling portion 21c via the held portion Wa. Each of the plurality of drive wires is coupled to a corresponding one of the plurality of coupling portions.

To be specific, the first held portion Wa11 of the first drive wire W11 is coupled to the first coupling portion 21c11. The second held portion Wa12 of the second drive wire W12 is coupled to the second coupling portion 21c12. The third held portion Wa13 of the third drive wire W13 is coupled to the third coupling portion 21c13. The fourth held portion Wa21 of the fourth drive wire W21 is coupled to the fourth coupling portion 21c21. The fifth held portion Wa22 of the fifth drive wire W22 is coupled to the fifth coupling portion 21c22. The sixth held portion Wa23 of the sixth drive wire W23 is coupled to the sixth coupling portion 21c23. The seventh held portion Wa31 of the seventh drive wire W31 is coupled to the seventh coupling portion 21c31. The eighth held portion Wa32 of the eighth drive wire W32 is coupled to the eighth coupling portion 21c32. The ninth held portion Wa33 of the ninth drive wire W33 is coupled to the ninth coupling portion 21c33.

The base unit 200 includes a base frame 25. The base frame 25 has a plurality of insertion holes respectively for inserting the first to ninth drive wires (W11 to W33). The base frame 25 has a first insertion hole 25a11, a second insertion hole 25a12, a third insertion hole 25a13, a fourth insertion hole 25a21, a fifth insertion hole 25a22, a sixth insertion hole 25a23, a seventh insertion hole 25a31, an eighth insertion hole 25a32, and a ninth insertion hole 25a33. The first to ninth insertion holes (25a11 to 25a33) respectively correspond to the first to ninth drive wires (W11 to W33). The numeral after the symbol 25a represents the numeral of the corresponding drive wire. For example, the first drive wire W11 is inserted into the first insertion hole 25a11.

Any one of the first to ninth insertion holes (25a11 to 25a33) can be referred to as an insertion hole 25a. In the present embodiment, the first to ninth insertion holes (25a11 to 25a33) have the same shape.

The base frame 25 has an attachment opening 25b into which the wire cover 14 is inserted. The first to ninth insertion holes (25a11 to 25a33) are arranged in a bottom portion of the attachment opening 25b.

Moreover, the base unit 200 includes a motor frame 200b, a first bearing frame 200c, a second bearing frame 200d, and a third bearing frame 200e. The motor frame 200b, the first bearing frame 200c, the second bearing frame 200d, and the third bearing frame 200e are coupled.

The base frame 25 includes the key receiving portion (key hole, base-side key, body-side key) 22 that receives the key shaft 15. As the key shaft 15 and the key receiving portion 22 engage with each other, the catheter unit 100 is prevented from being attached to the base unit 200 with a wrong phase.

As the key shaft 15 and the key receiving portion 22 engage with each other, with respect to the circumferential direction of the circle (imaginary circle) along which the first to ninth drive wires (W11 to W33) are arranged, movement of the catheter unit 100 relative to the base unit 200 is limited in a predetermined range.

As a result, each of the first to ninth drive wires (W11 to W33) engages with a corresponding one of the first to ninth insertion holes (25a11 to 25a33) and a corresponding one of the first to ninth coupling portions (21c11 to 21c33). In other words, the drive wire W is prevented from engaging with an insertion hole 25a different from a corresponding insertion hole 25a and with 21c different from a corresponding coupling portion 21c.

A user can correctly couple each of the first to ninth drive wires (W11 to W33) to a corresponding one of the first to ninth coupling portions (21c11 to 21c33) by engaging the key shaft 15 with the key receiving portion 22. Accordingly, a user can easily attach the catheter unit 100 to the base unit 200.

In the present embodiment, the key shaft 15 has a protruding portion protruding in a direction that intersects the attachment/detachment directions DE and the key receiving portion 22 has a recessed portion into which the protruding portion is inserted. In the circumferential direction, a position where the protruding portion and the recessed portion engage with each other is a position where the drive wire W engages with a corresponding insertion hole 25a and a corresponding coupling portion 21c.

### <Coupling of Drive Source and Drive Wire>

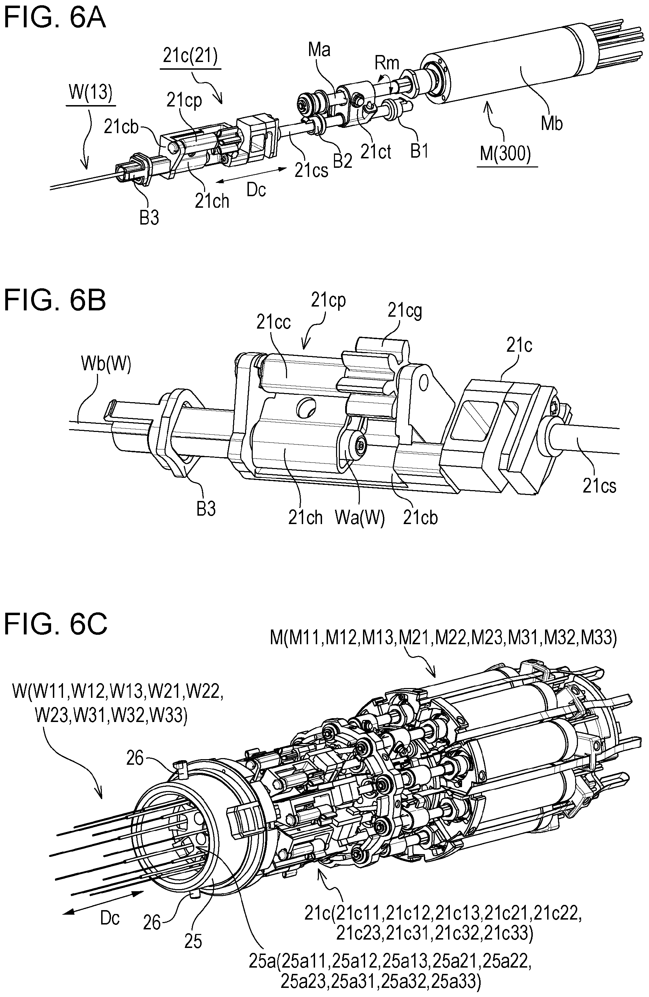

Referring to Figs. 6A to 6C, coupling of the wire driving portion 300, the coupling device 21, and the bending driving portion 13 will be described. Figs. 6A to 6C are views illustrating the wire driving portion 300, the coupling device 21, and the bending driving portion 13.

Fig. 6A is a perspective view of the drive source M, the coupling portion 21c, and the drive wire W. Fig. 6B is an enlarged view of the coupling portion 21c and the drive wire W. Fig. 6C is a perspective view illustrating coupling of the wire driving portion 300, the coupling device 21, and the bending driving portion 13.

In the present embodiment, configurations with which the first to ninth drive wires (W11 to W33) and the first to ninth coupling portions (21c11 to 21c33) are respectively coupled are the same. Configurations with which the first to ninth coupling portions (21c11 to 21c33) and the first to ninth drive sources (M11 to M33) are respectively connected are the same. Accordingly, in the following description, by using one drive wire W, one coupling portion 21c, and one drive source M, configuration with which these are connected will be described.

As illustrated in Fig. 6A, the drive source M includes a motor shaft Ma and a motor body Mb that rotates the motor shaft Ma in rotation directions Rm. A helical groove is formed in a surface of the motor shaft Ma. The motor shaft Ma has a so-called screw shape. The motor body Mb is fixed to the motor frame 200b.

The coupling portion 21c includes a tractor 21ct that is connected to the motor shaft Ma and a tractor supporting shaft 21cs that supports the tractor 21ct. The tractor supporting shaft 21cs is connected to a coupling base 21cb via a force sensor 21cf. The force sensor 21cf converts a tension that the drive wire W receives into an electric signal, and outputs the electric signal to the controller 3. Details will be described below in the sixth embodiment.

The coupling portion 21c includes a plate spring 21ch as a holding portion for holding the held portion Wa of the drive wire W. The drive wire W extends through the insertion hole 25a and is engaged with the coupling portion 21c. To be more specific, the held portion Wa engages with the plate spring 21ch. As described below, the plate spring 21ch can enter a state (fixing state) in which the plate spring 21ch clips and fixes the held portion Wa and a state (releasing state) in which the plate spring 21ch releases the held portion Wa.

The coupling portion 21c includes a pressing member 21cp. The pressing member 21cp includes a gear portion 21cg that meshes with an internal gear 29 described below and a cam 21cc as a pressing portion for pressing the plate spring 21ch.

As described below, the cam 21cc can move relative to the plate spring 21ch. As the cam 21cc moves, the fixing state and the releasing state of the plate spring 21ch are switched.

The coupling portion 21c is supported by a first bearing B1, a second bearing B2, and a third bearing B3. The first bearing B1 is supported by the first bearing frame 200c of the base unit 200. The second bearing B2 is supported by the second bearing frame 200d of the base unit 200. The third bearing B3 is supported by the third bearing frame 200e of the base unit 200. Accordingly, when the motor shaft Ma rotates in the rotation directions Rm, the coupling portion 21c is restrained from rotating around the motor shaft Ma. The first bearing B1, the second bearing B2, and the third bearing B3 are provided for each of the first to ninth coupling portions (21c11 to 21c33).

Since the coupling portion 21c is restrained from rotating around the motor shaft Ma, when the motor shaft Ma rotates, due to the helical groove of the motor shaft Ma, a force in the rotation-axis direction of the motor shaft Ma acts on the tractor 21ct. As a result, the coupling portion 21c moves along the rotation-axis directions of the motor shaft Ma (the Dc directions). As the coupling portion 21c moves, the drive wire W also moves in the Dc directions, and the bending portion 12 bends. That is, the motor shaft Ma and the tractor 21ct constitute a so-called feed screw that converts rotational movement transmitted from the drive source M into linear movement by using a screw.

As illustrated in Fig. 6C, when the catheter unit 100 is attached to the base unit 200, the first to ninth drive wires (W11 to W33) are respectively coupled to the first to ninth coupling portions (21c11 to 21c33).

The controller 3 can control the first to ninth drive sources (M11 to M33) independently from each other. That is, any of the first to ninth drive sources (M11 to M33) can independently operate or stop irrespective of whether the other drive sources are in a stopped state or not. In other words, the controller 3 can control the first to ninth drive wires (W11 to W33) independently from each other. As a result, the first to third guide rings (J1 to J3) are controlled independently from each other, and the bending region 12b of the bending portion 12 can bend in any direction.

To be more specific, the third guide ring J3 is provided at the distal end of the bending portion 12. The seventh to ninth drive wires (W31 to W33) are connected to the third guide ring J3, and the seventh to ninth drive sources (M31 to M33) are connected to these three drive wires. That is, by controlling these three drive sources (M31 to M33), it is possible to change the orientation of the third guide ring in any direction, and the bending region 12b bends on an arc along the direction. Likewise, by controlling the fourth to sixth drive sources (M21 to M23), it is possible to change the orientation of the second guide ring J2 in any direction. Likewise, by controlling the first to third drive sources (M11 to M13), it is possible to change the orientation of the first guide ring J1 in any direction.

Since the three guide rings (J1 to J3) can face in any directions independently from each other as described above, the bending region 12b can have a complex shape. Thus, the catheter 11 can be inserted into and moved in an organ in the body of a patient having an elongated and complex shape, such as a bronchus or a digestive organ.

### <Attachment of Catheter Unit>

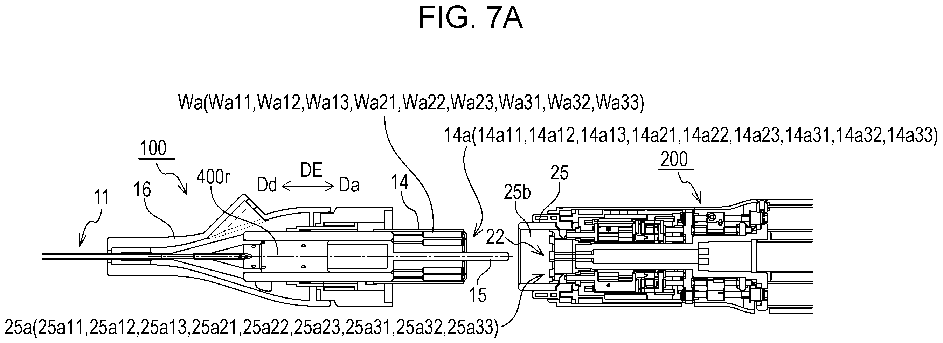

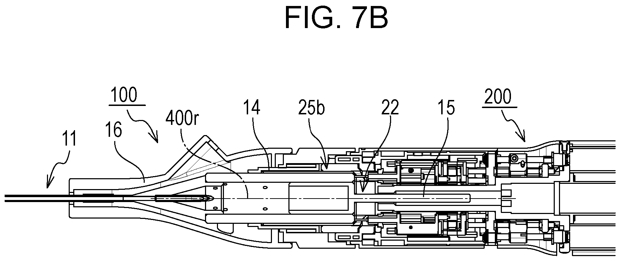

Referring to Figs. 7A and 7B, an operation of attaching the catheter unit 100 to the base unit 200 will be described.

Figs. 7A and 7B are views illustrating attachment of the catheter unit 100. Fig. 7A is a view illustrating a state before the catheter unit 100 is attached to the base unit 200. Fig. 7B is a view illustrating a state after the catheter unit 100 has been attached to the base unit 200.

In the present embodiment, the attachment/detachment directions DE of the catheter unit 100 are the same as the directions of the rotation axis 400r of the operating portion 400. Among the attachment/detachment directions DE, a direction in which the catheter unit 100 is attached to the base unit 200 will be referred to as an attachment direction Da. Among the attachment/detachment directions DE, a direction in which the catheter unit 100 is detached from the base unit 200 (a direction opposite to the attachment direction Da) will be referred to as a detachment direction Dd.

As illustrated in Fig. 7A, in a state before the catheter unit 100 is attached to the base unit 200, the wire cover 14 is located at the covering position. At this time, the wire cover 14 covers the first to ninth drive wires (W11 to W33) so that the first to ninth held portions (Wall to Wa33) may not protrude from the first to ninth exposure holes (14a11 to 14a33) of the wire cover 14. Accordingly, in the state before the catheter unit 100 is attached to the base unit 200, the first to ninth drive wires (W11 to W33) can be protected.

When the catheter unit 100 is to be attached to the base unit 200, the key shaft 15 is made to engage with the key receiving portion 22. The key shaft 15 protrudes from the wire cover 14. In the present embodiment, in a state in which the key shaft 15 has reached the inlet of the key receiving portion 22, the wire cover 14 does not engage with the attachment opening 25b. That is, when the phase of the catheter unit 100 relative to the base unit 200 is a phase that does not allow the key shaft 15 and the key receiving portion 22 to engage with each other, the wire cover 14 does not engage with the attachment opening 25b, and a state in which the wire cover 14 is located at the covering position is maintained. Accordingly, even when the catheter unit 100 is moved so that the key shaft 15 and the key receiving portion 22 engage with each other, the first to ninth drive wires (W11 to W33) are protected.

When the key shaft 15 and the key receiving portion 22 engage with each other and the catheter unit 100 is moved in the attachment direction Da relative to the base unit 200, the catheter unit 100 is attached to the base unit 200. As the catheter unit 100 is attached to the base unit 200, the wire cover 14 moves to the exposing position. In the present embodiment, the wire cover 14 moves from the covering position to the exposing position by contacting the base frame 25 (see Fig. 7B).

To be more specific, when the catheter unit 100 is being attached, the wire cover 14 contacts the base frame 25 and stops. As the catheter unit 100 is moved in the attachment direction Da in this state, in the catheter unit 100, the wire cover 14 moves relative to portions other than the wire cover 14. As a result, the wire cover 14 moves from the covering position to the exposing position.

While the wire cover 14 moves from the covering position to the exposing position, the held portion Wa of the drive wire W protrudes from the exposure hole 14a of the wire cover 14 and is inserted into the insertion hole 25a. Then, the held portion Wa engages with the plate spring 21ch of the coupling portion 21c (see Fig. 6B).

In a state in which the catheter unit 100 is only attached to the base unit 200, it is possible to detach the catheter unit 100 by moving the catheter unit 100 in the detachment direction Dd relative to the base unit 200. As described below, in the state in which the catheter unit 100 is only attached to the base unit 200, fixing of the drive wire W and the coupling portion 21c is in a state of being released.

By operating the operating portion 400 in the state in which the catheter unit 100 is attached to the base unit 200, detachment of the catheter unit 100 from the base unit 200 is prevented. Moreover, by operating the operating portion 400 in the state in which the catheter unit 100 is attached to the base unit 200, the bending driving portion 13 is fixed to the coupling device 21, and the bending driving portion 13 is coupled to the wire driving portion 300 via the coupling device 21.

### <Fixing and Releasing of Fixing of Bending Driving Portion>

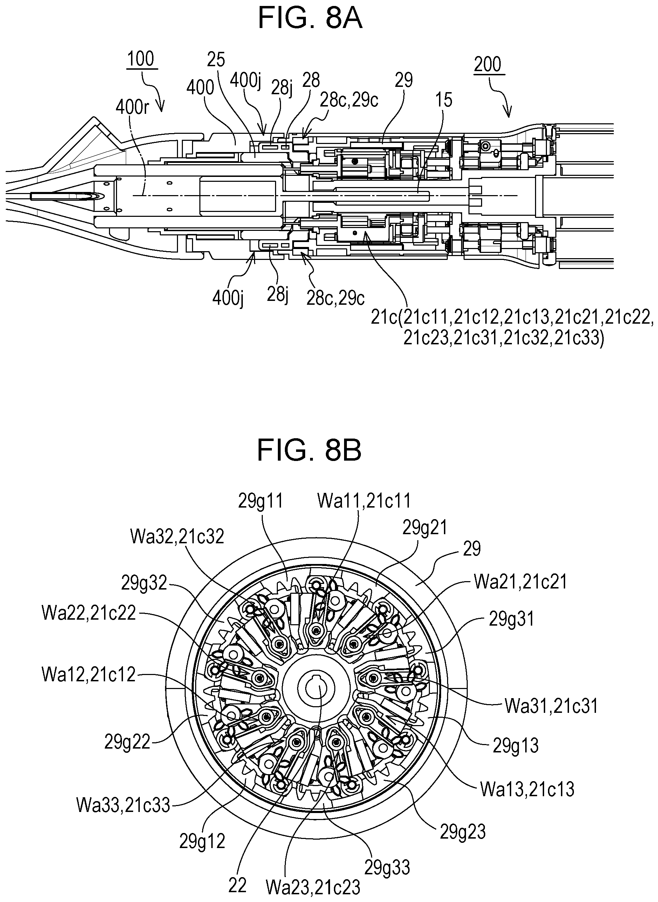

Referring to Figs. 8A, 8B, 9, 10, 11, 12, 13, and 14, a configuration for fixing the bending driving portion 13 to the coupling device 21 and a configuration for releasing fixing of the bending driving portion 13 by the coupling device 21 will be described.

Figs. 8A and 8B are views illustrating coupling of the catheter unit 100 and the base unit 200. Fig. 8A is a sectional view of the catheter unit 100 and the base unit 200. Fig. 8A is a sectional view of the catheter unit 100 and the base unit 200 cut along the rotation axis 400r. Fig. 8B is a sectional view of the base unit 200. This is a sectional view of the base unit 200 cut at the coupling portion 21c in a direction perpendicular to the rotation axis 400r.

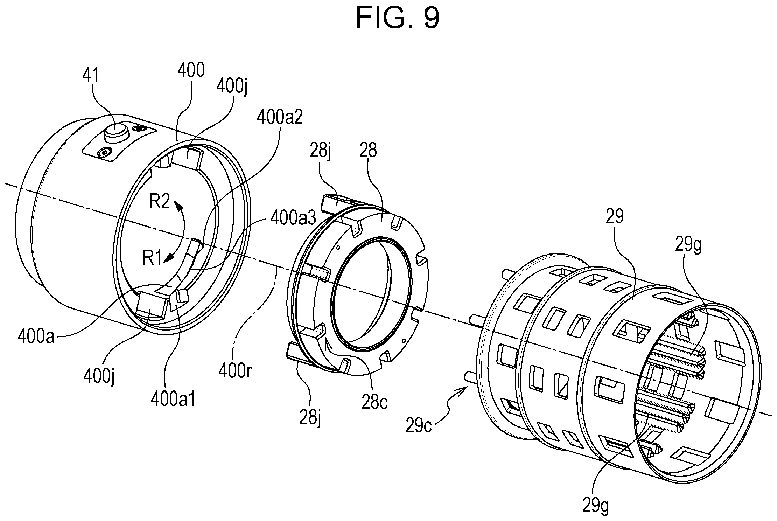

Fig. 9 is an exploded view illustrating coupling of the catheter unit 100 and the base unit 200.

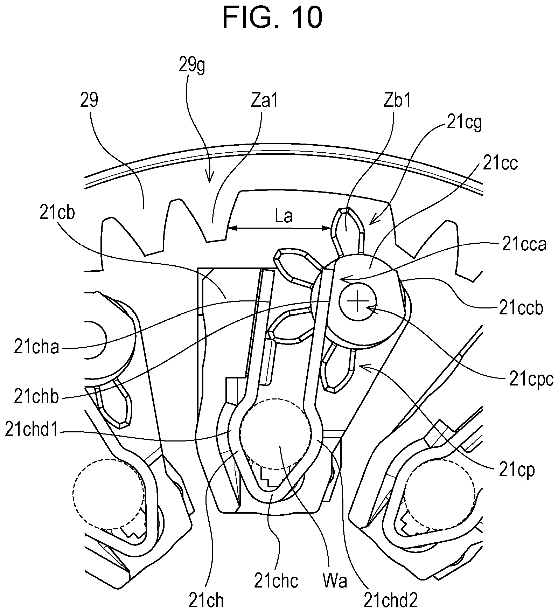

Figs. 10, 11, 12, 13, and 14 are views illustrating fixing of the drive wire W by the coupling portion 21c.

As illustrated in Figs. 8A and 9, the base unit 200 includes a joint (middle member, second transmitting member) 28 and the internal gear 29 as a movement gear (conjunctive movement gear, transmitting member, first transmitting member) that moves in conjunction with the operating portion 400 via the joint 28.

The joint 28 includes a plurality of transmitting portions 28c, and the internal gear 29 includes a plurality of transmitted portions 29c. The plurality of transmitting portions 28c are engaged with the plurality of transmitting portions 29c, and, when the joint 28 rotates, rotation of the joint 28 is transmitted to the internal gear 29.

When the catheter unit 100 is attached to the base unit 200, an engagement portion 400j of the operating portion 400 engages with a joint engagement portion 28j of the joint 28. When the operating portion 400 rotates, rotation of the operating portion 400 is transmitted to the joint 28. The operating portion 400, the joint 28, and the internal gear 29 rotate in the same direction.

The internal gear 29 includes a plurality of tooth portions for switching between a state in which each of the first to ninth coupling portions (21c11 to 21c33) fixes a corresponding one of the first to ninth drive wires (W11 to W33) and a state in which each of the first to ninth coupling portions (21c11 to 21c33) releases a corresponding one of the first to ninth drive wires (W11 to W33). Each of the plurality of tooth portions (action portions, switching gear portions) of the internal gear 29 engages with the gear portion 21cg of the pressing member 21cp of a corresponding one of the first to ninth coupling portions (21c11 to 21c33).

To be specific, in the present embodiment, the internal gear 29 includes a first tooth portion 29g11, a second tooth portion 29g12, a third tooth portion 29g13, a fourth tooth portion 29g21, a fifth tooth portion 29g22, a sixth tooth portion 29g23, a seventh tooth portion 29g31, an eighth tooth portion 29g32, and a ninth tooth portion 29g33. The first to ninth tooth portions (29g11 to 29g33) are formed with a gap between each other.

The first tooth portion 29g11 meshes with the gear portion 21cg of the first coupling portion 21c11. The second tooth portion 29g12 meshes with the gear portion 21cg of the second coupling portion 21c12. The third tooth portion 29g13 meshes with the gear portion 21cg of the third coupling portion 21c13. The fourth tooth portion 29g21 meshes with the gear portion 21cg of the fourth coupling portion 21c21. The fifth tooth portion 29g22 meshes with the gear portion 21cg of the fifth coupling portion 21c22. The sixth tooth portion 29g23 meshes with the gear portion 21cg of the sixth coupling portion 21c23. The seventh tooth portion 29g31 meshes with the gear portion 21cg of the seventh coupling portion 21c31. The eighth tooth portion 29g32 meshes with the gear portion 21cg of the eighth coupling portion 21c32. The ninth tooth portion 29g33 meshes with the gear portion 21cg of the ninth coupling portion 21c33.

Any one of the first to ninth tooth portions (29g11 to 29g33) can be referred to as a tooth portion 29g. In the present embodiment, the first to ninth tooth portions (29g11 to 29g33) have the same configuration.

In the present embodiment, configurations with which the first to ninth drive wires (W11 to W33) and the first to ninth coupling portions (21c11 to 21c33) are respectively coupled are the same. Configurations with which the first to ninth coupling portions (21c11 to 21c33) and the first to ninth tooth portions (29g11 to 29g33) are respectively connected are the same. Accordingly, in the following description, by using one drive wire W, one coupling portion 21c, and one tooth portion 29g, configuration with which these are connected will be described.

In each of the first to ninth coupling portions (21c11 to 21c33), as the gear portion 21cg is moved by the internal gear 29, the pressing member 21cp rotates, and the cam 21cc moves to a pressed position and a retracted position retracted from the pressed position.

As the operating portion 400 is rotated, the internal gear 29 rotates. As the internal gear 29 rotates, each of the first to ninth coupling portions (21c11 to 21c33) operates. That is, by performing an operation of rotating one operating portion 400, it is possible to cause the first to ninth coupling portions (21c11 to 21c33) to operate.

In a state in which the catheter unit 100 is attached to the base unit 200, the operating portion 400 can move to a fixing position (locking position) and a detachment position. As described below, in the state in which the catheter unit 100 is attached to the base unit 200, the operating portion 400 can move to a release position. In the circumferential direction of the operating portion 400, the release position is located between the fixing position and the detachment position. In a state in which the operating portion 400 is located at the detachment position, the catheter unit 100 is attached to the base unit 200.

In the state in which the catheter unit 100 is attached to the base unit 200, fixing (locking) of the drive wire W to the coupling portion 21c is in a state of being released. This state will be referred to as a releasing state of the coupling portion 21c. A state in which the drive wire W is fixed (locked) to the coupling portion 21c will be referred to as a locking state of the coupling portion 21c.

Referring to Figs. 10, 11, 12, 13, and 14, an operation when fixing the drive wire W to the coupling portion 21c will be described.

In a state after the catheter unit 100 has been attached to the base unit 200 and before the operating portion 400 is operated, the catheter unit 100 can be detached from the base unit 200. Hereafter, a state in which it is possible to detach the catheter unit 100 from the base unit 200 will be referred to as a detachable state.

Fig. 10 illustrates a state of the internal gear 29 and the coupling portion 21c in the detachable state. Fig. 10 illustrates the internal gear 29 and the coupling portion 21c in a state in which the operating portion 400 is located at the fixing position.

The plate spring 21ch of the coupling portion 21c includes a fixed portion 21cha that is fixed to the coupling base 21cb and a pressed portion 21chb that contacts the cam 21cc of the pressing member 21cp. The plate spring 21ch includes a first portion 21chd1 and a second portion 21chd2. When the catheter unit 100 is attached to the base unit 200, the held portion Wa is inserted between the first portion 21chd1 and the second portion chd2.

The cam 21cc has a holding surface 21cca and a pressing surface 21ccb. With respect to the radial direction of the pressing member 21cp, the holding surface 21cca is disposed at a position closer than the pressing surface 21ccb to a rotation center 21cpc of the pressing member 21cp.

As illustrated in Fig. 10, in the detachable state (a state in which the operating portion 400 is located at the detachment position), the plate spring 21ch is held at a position where the pressed portion 21chb is in contact with the holding surface 21cca. A tooth Za1 of the internal gear 29 and a tooth Zb1 of the gear portion 21cg are stopped in a state in which a clearance La is generated therebetween.

In the rotation directions of the operating portion 400, a direction in which the operating portion 400 moves from the detachment position toward the release position and the fixing position will be referred to as a locking direction (fixing direction), and a direction in which the operating portion 400 moves from the fixing position toward the release position and the detachment position will be referred to as a releasing direction. The operating portion 400 rotates from the release position in the releasing direction to move to the detachment position. The operating portion 400 rotates from the release position in the locking direction to move to the fixing position.

In a state in which the catheter unit 100 is attached to the base unit 200 and the operating portion 400 is at the detachment position, the coupling portion 21c is in the releasing state, which is a state in which fixing of the drive wire W by the coupling portion 21c is released.

When the coupling portion 21c is in the releasing state, the cam 21cc is located at the retracted position retracted from the pressed position described below. At this time, fixing of the held portion Wa by the plate spring 21ch is in a state of being released. A force with which the first portion 21chd1 and the second portion 21chd2 clamp the held portion Wa when the coupling portion 21c is in the releasing state is smaller than a force with which the first portion 21chd1 and the second portion 21chd2 clamp the held portion Wa when the coupling portion 21c is in the locking state.

When the coupling portion 21c is in the releasing state, if the catheter unit is moved in the detachment direction Dd relative to the base unit 200, the held portion Wa can be pulled out from between the first portion 21chd1 and the second portion 21chd2.

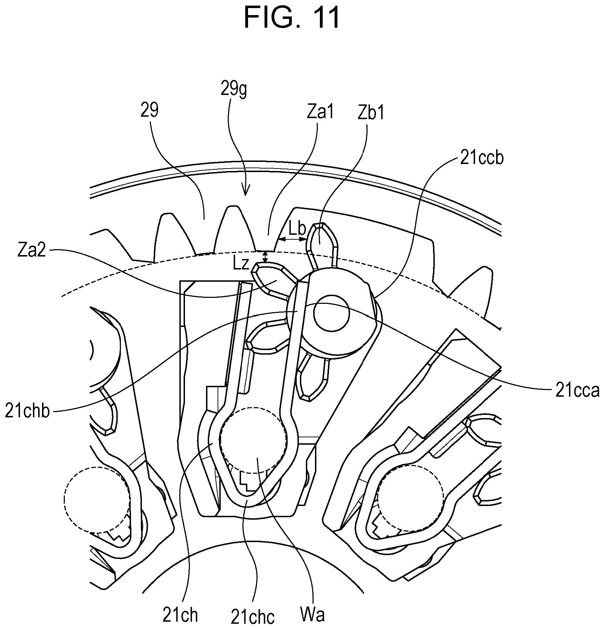

Fig. 11 is a view illustrating the state of the internal gear 29 and the coupling portion 21c when the operating portion 400 is rotated from the detachment position in the locking direction. Fig. 11 is a view illustrating the state of the internal gear 29 and the coupling portion 21c in a state in which the operating portion 400 is at the release position.

In a state in which the operating portion 400 is at the detachment position (Fig. 10), when the operating portion 400 is rotated in the locking direction, the internal gear 29 rotates clockwise. Then, the operating portion 400 is located at the release position.

Even when the operating portion 400 is rotated, since the key shaft 15 and the key receiving portion 22 are engaged with each other, the entirety of the catheter unit 100 (excluding the operating portion 400) is restrained from rotating relative to the base unit 200. That is, in a state in which the entirety of the catheter unit 100 (excluding the operating portion 400) and the base unit 200 are stopped, the operating portion 400 is rotatable relative to these.

As the internal gear 29 rotates clockwise, the clearance between the tooth Za1 of the internal gear 29 and the tooth Zb1 of the gear portion 21cg decreases from the clearance La to a clearance Lb.

The tooth Zb2 of the gear portion 21cg is disposed at a position separated by a clearance Lz from the addendum circle (dotted line) of the tooth portion 29g of the internal gear 29. Therefore, the internal gear 29 is rotatable without interfering with the tooth Zb2. On the other hand, the coupling portion 21c is maintained in a state (releasing state) that is the same as the state illustrated in Fig. 10.

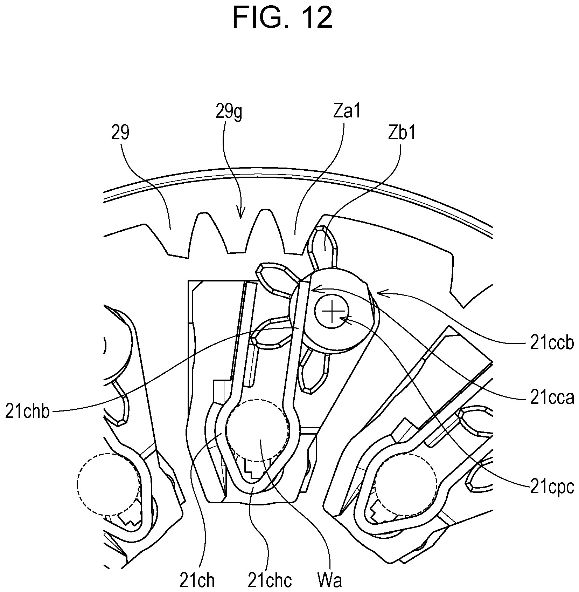

When the operating portion 400 is further rotated in the locking direction from the state illustrated in Fig. 11, the internal gear 29 further rotates clockwise. The state of the internal gear 29 and the coupling portion 21c at this time is illustrated in Fig. 12.

Fig. 12 is a view illustrating the state of the internal gear 29 and the coupling portion 21c when the operating portion 400 is rotated from the release position in the locking direction.

As illustrated in Fig. 12, when the operating portion 400 is rotated from the release position in the locking direction, the tooth Za1 of the internal gear 29 and the tooth Zb1 of the gear portion 21cg contact each other. On the other hand, the coupling portion 21c is in the same state as the state illustrated in Figs. 10 and 11, and is maintained in the releasing state.

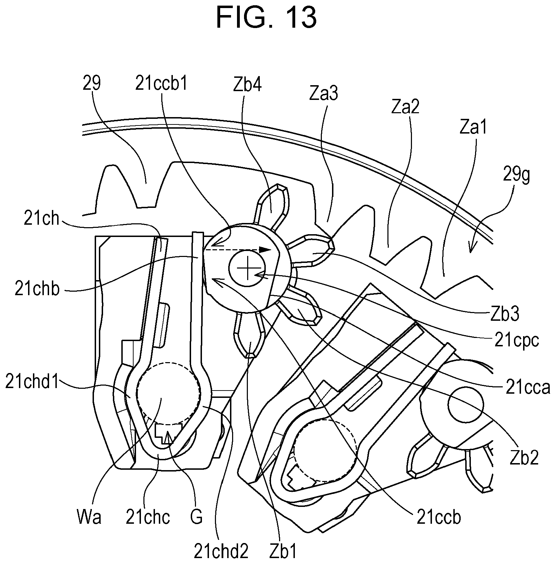

Fig. 13 is a view illustrating a state in which the pressing member 21cp has rotated as the operating portion 400 has rotated in the locking direction.

As illustrated in Fig. 13, when the operating portion 400 is further rotated in the locking direction from the state illustrated in Fig. 12, the internal gear 29 further rotates clockwise.

As the internal gear 29 moves from the state illustrated in Fig. 12 to the state illustrated in Fig. 13, the internal gear 29 moves the gear portion 21cg clockwise. When the gear portion 21cg rotates, the holding surface 21cca becomes separated from the pressed portion 21chb, and the pressing surface 21ccb becomes closer to the pressed portion 21chb. Then, clipping of the held portion Wa by the first portion 21chd1 and the second portion 21chd2 is started.

Then, while the pressed portion 21chb is pressed by a corner portion 21ccb1 disposed at an end portion of the pressing surface 21ccb, a tooth Za3 of the internal gear 29 moves to a position separating from a tooth Zb3 of the gear portion 21cg. At this time, the held portion Wa is in a state of being clipped between the first portion 21chd1 and the second portion 21chd2.

When the tooth Za3 of the internal gear 29 becomes separated from the tooth Zb3 of the gear portion 21cg, transmission of a driving force from the internal gear 29 to the gear portion 21cg finishes. At this time, the cam 21cc is in a state such that the corner portion 21ccb1 receives a reaction force from the plate spring 21ch.

In the radial direction of the pressing member 21cp, the reaction force from the plate spring 21ch, which acts on the corner portion 21ccb1, acts at a position that is separated from the rotation center 21cpc of the pressing member 21cp, and the pressing member 21cp rotates clockwise. At this time, the pressing member 21cp rotates in the same direction as the direction in which the pressing member 21cp is rotated by the internal gear 29 that rotates clockwise.

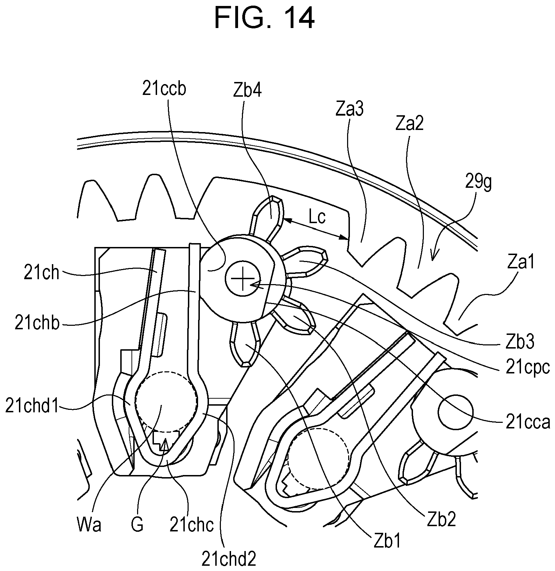

Fig. 14 is a view illustrating the internal gear 29 and the coupling portion 21c in a state in which the operating portion 400 is at the fixing position.

As illustrated in Fig. 14, from the state illustrated in Fig. 13, the pressing member 21cp further rotates by receiving the reaction force of the plate spring 21ch.

As illustrated in Fig. 14, the pressing member 21cp stops in a state in which the pressing surface 21ccb of the cam 21cc and the pressed portion 21chb of the plate spring 21ch are in surface-contact with each other. That is, the pressing surface 21ccb and the surface of the pressed portion 21chb are in a state of being arranged on the same plane.

At this time, the coupling portion 21c is in the locking state. When the coupling portion 21c is in the locking state, the cam 21cc of the pressing member 21cp is located at the pressed position, and the pressing surface 21ccb presses the pressed portion 21chb.

When the coupling portion 21c is in the locking state, the held portion Wa is clipped between the first portion 21chd1 and the second portion 21chd2. That is, the plate spring 21ch is pressed by the cam 21cc, and the held portion Wa is clamped by the plate spring 21ch. As a result, the held portion Wa is fixed by the plate spring 21ch.

In the present embodiment, the first portion 21chd1 and the second portion 21chd2 of the plate spring 21ch press the held portion Wa at positions separated from each other. Moreover, a bent portion 21chc that links the first portion 21chd1 and the second portion 21chd2 is disposed between the first portion 21chd1 and the second portion 21chd2. The bent portion 21chc is disposed so as to be separated by a gap G from the held portion Wa. By doing so, it is possible to stably fix the held portion Wa by using the first portion 21chd1 and the second portion 21chd2.

When the coupling portion 21c is in the locking state, pulling out of the held portion Wa from between the first portion 21chd1 and the second portion 21chd2 is limited.

The tooth Za3 of the internal gear 29 and a tooth Zb4 of the gear portion 21cg stop at positions such that a clearance Lc is generated therebetween.

When fixing of the drive wire W and the coupling portion 21c is to be released, the operating portion 400 located at the fixing position is rotated in the releasing direction. At this time, the internal gear 29 rotates counterclockwise from the state illustrated in Fig. 14. When the internal gear 29 rotates counterclockwise, the tooth Za3 of the internal gear 29 contacts the tooth Zb4 of the gear portion 21cg, and the pressing member 21cp is rotated counterclockwise.

As the internal gear 29 further rotates counterclockwise, fixing of the drive wire W by the coupling portion 21c is released. The operation of the internal gear 29 and the pressing member 21cp at this time is opposite to the operation described above. That is, due to an operation opposite to the operation described above that is performed when the drive wire W is to be fixed by the coupling portion 21c, fixing of the drive wire W by the coupling portion 21c is released.

The operation described above is performed in each of the first to ninth coupling portions (21c11 to 21c33). That is, in the process through which the operating portion 400 moves from the detachment position to the fixing position, due to movement (rotation) of the operating portion 400, the state of the first to ninth coupling portions (21c11 to 21c33) changes from the releasing state to the locking state. In the process through which the operating portion 400 moves from the fixing position to the detachment position, due to movement (rotation) of the operating portion 400, the state of the first to ninth coupling portions (21c11 to 21c33) changes from the locking state to the releasing state. That is, a user can switch a plurality of coupling portions between the releasing state and the locking state by operating one operating portion 400.

That is, it is not necessary that an operating portion for switching between the releasing state and the locking state be provided in each of the plurality of coupling portions and that a user operate the operating portion. Accordingly, a user can easy attach/detach the catheter unit 100 to/from the base unit 200.

A state in which each of the first to ninth drive wires (W11 to W33) is fixed by a corresponding one of the first to ninth coupling portions (21c11 to 21c33) will be referred to as a first state. A state in which fixing of each of the first to ninth drive wires (W11 to W33) by a corresponding one of the first to ninth coupling portions (21c11 to 21c33) is released will be referred to as a second state.

The first state and the second state are switched in conjunction with movement of the operating portion 400. That is, the first state and the second state are switched in conjunction of movement of the operating portion 400 between the detachment position and the fixing position.

The internal gear 29 is configured to move in conjunction with the operating portion 400. In the present embodiment, the joint 28 functions as a transmission member for causing the operating portion 400 and the internal gear 29 to move in conjunction with each other. The internal gear 29 and the joint 28 have a function of a conjunctive-movement member that moves in conjunction with the operating portion 400 so that the first state and the second state are switched in conjunction with movement of the operating portion 400.

To be specific, in the state in which the catheter unit 100 is attached to the base unit 200, in conjunction with movement of the operating portion 400, the internal gear 29 and the joint 28 move a part of the plate spring 21ch (the pressed portion 21chb) relative to the held portion Wa. As the pressed portion 21chb moves, the locking state and the releasing state of the coupling portion 21c are switched.

### <Description of Information Storage>

The catheter unit 100 includes an information storage 110 as a first information storage that stores information about the catheter unit 100 (first information). When the catheter unit 100 is attached to the base unit 200, the controller 3 controls the wire driving portion 300 based on the first information acquired from the information storage 110.

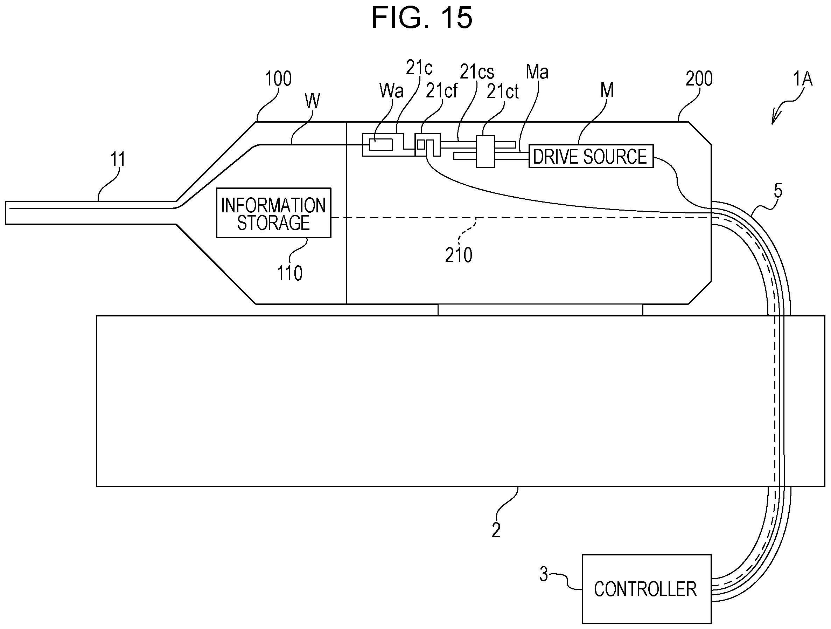

Fig. 15 is a schematic block diagram of the medical system 1A according to the present embodiment. When the catheter unit 100 is attached to the base unit 200, the controller 3 and the information storage 110 become connected by information communication means 210 shown by a dotted line in the figure, and it becomes possible for the controller 3 to acquire information stored in the information storage 110. It is sufficient that the information storage 110 include any means that can store information, such as a non-volatile memory, an RFID, or a bar code. As the information communication means 210, a unit having a configuration optimum for the means for realizing the information storage 110 is used, such as a communication line for a non-volatile memory, a communication antenna and a communication line for an RFID, or a bar-code reader and a communication line for a bar code.

In the present embodiment, a method of communication between the controller 3 and the information storage 110 may be either wired or wireless. In a case of wired communication, communication is performed in a state in which the catheter unit 100 is attached to the base unit 200. In a case of wireless communication, communication is possible even before the catheter unit 100 is attached to the base unit 200.

In a case of wired communication, an electric contact and a communication interface for exchanging information are provided in each of the catheter unit 100 and the base unit 200.

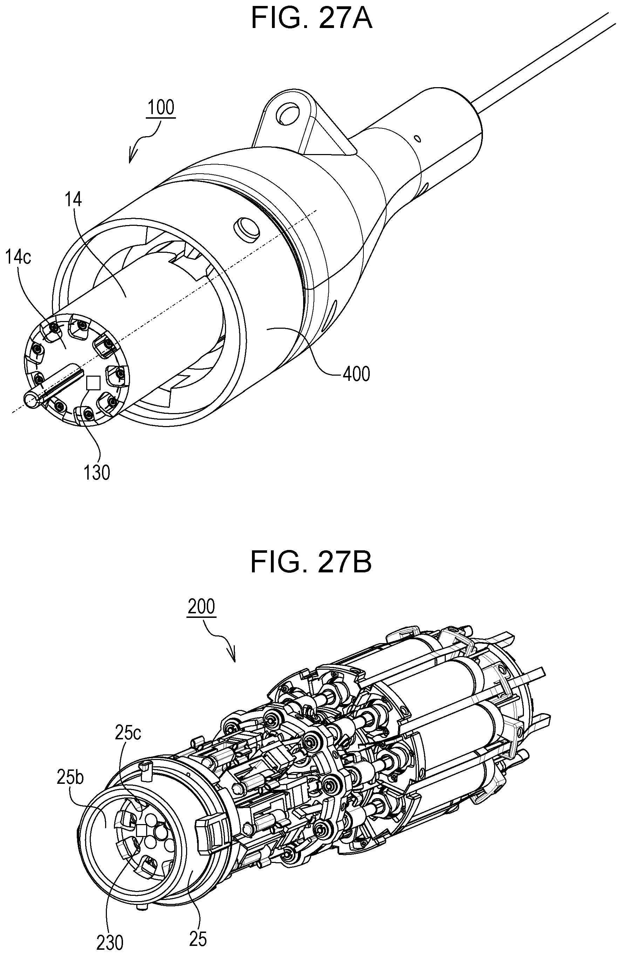

As an example, Fig. 27A illustrates a catheter-side electric contact 130 provided in the catheter unit 100, and Fig. 27B illustrates a base-unit-side electric contact 230 provided in the base unit 200.

As illustrated in Fig. 27A, the catheter-side electric contact 130 can be provided, for example, on a bottom surface 14c of the wire cover 14. The position of the catheter-side electric contact 130 is not limited to this, and instead, the catheter-side electric contact 130 may be provided, for example, on an outer peripheral surface of the wire cover 14 or on an inner peripheral surface of the operating portion 400.

As illustrated in Fig. 27B, the base-unit-side electric contact 230 may be provided, for example, on an inner bottom surface 25c of the base frame 25. The position of the base-unit-side electric contact 230 is not limited to this, and instead, the base-unit-side electric contact 230 may be provided, for example, on an inner peripheral surface (the attachment opening 25b) of the base frame 25 or on an outer peripheral surface of the base frame 25.

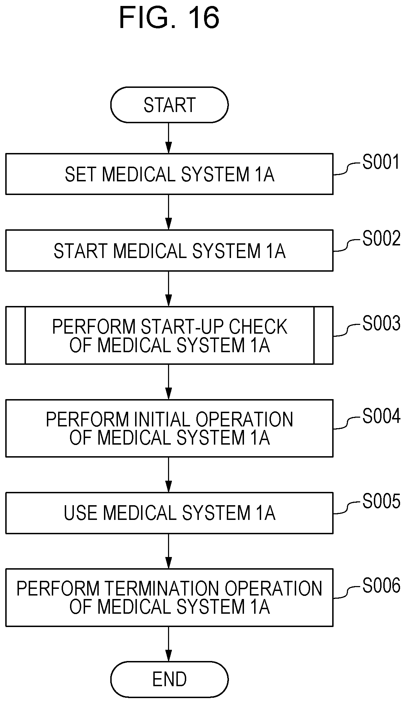

The medical system 1A is used in accordance with the procedure illustrated in Fig. 16. A user sets the medical system 1A at a position where it is possible to insert the catheter 11 to the inside of a subject (such as the oral cavity of the nasal cavity of a patient) (S001).

After the medical system 1A has been set, the user switches on the medical system 1A to start the controller 3 (S002).

After having been started, the controller 3 performs a start-up check for using the medical device 1 (S003). In the start-up check (S003), the catheter unit 100 is attached to the base unit 200, and acquisition of information from the information storage 110 and an operation based thereon are performed. This processing will be described below in detail.

After the start-up check (S003) has finished, an initial operation is performed (S004). In the initial operation (S004), works and processing that are necessary for using the medical system 1A are performed. To be specific, condition checks and operation checks of sensors, units, and software that are necessary for using the medical system 1A are performed.

When preparations necessary for using the medical system 1A are completed in the initial operation (S004), the user operates the medical device 1 and inserts the catheter 11 to the inside of a subject. Thus, works such as observation of the inside of the subject, sampling of various specimens from the inside of the subject, and treatment of the inside of the subject are performed (S005).

When the works by the user have been finished, the user performs a termination operation for terminating the medical system 1A (S006). In the termination operation (S006), the catheter unit 100 is detached from the base unit 200, and works and processing that are necessary for terminating the medical system 1A are performed. To be specific, condition checks and operation checks of sensors, units, and software that are necessary for terminating the medical system 1A are performed.

### <Control based on Historical Information>

As described above, the catheter unit 100 is attachable to and detachable from the base unit 200. In view of hygiene, it is preferable that the catheter unit 100, which is to be inserted to the inside of a subject, be replaced for each operation or manipulation.

The present embodiment is characterized in that the information storage 110 stores historical information about whether the catheter unit has been used or has not been used, and the operation of the medical device 1 is determined based on the information. That is, the first information includes the historical information.

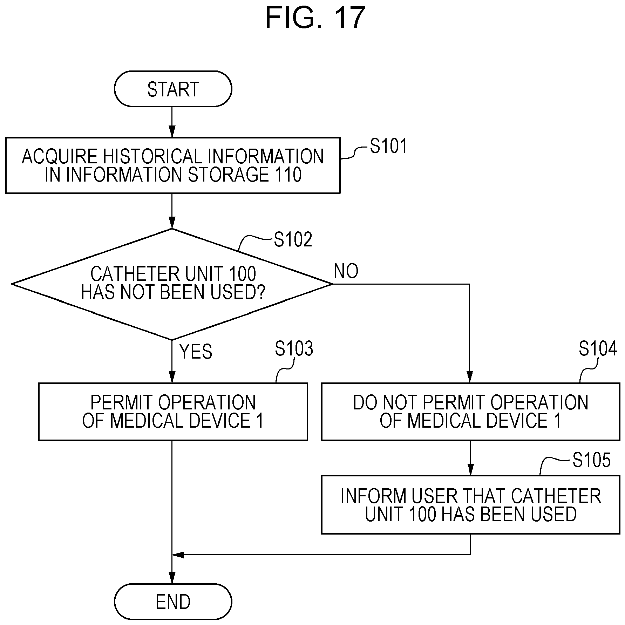

When the catheter unit 100 is manufactured, historical information indicating that the catheter unit 100 has not been used is stored in the information storage 110. Fig. 17 illustrates a flowchart of control according to the present embodiment. The flowchart illustrated in Fig. 17 is realized as a CPU (not shown) included in the controller 3 executes a program stored in a ROM (not shown). The present control corresponds to the start-up check (S003) in the procedure for using the medical system 1A illustrated in Fig. 16.

The controller 3 acquires the historical information stored in the information storage 110 (S101), and determines from the historical information whether or not the catheter unit 100 has not been used (S102). If the historical information indicates that the catheter unit 100 has not been used (YES in S102), the controller 3 determines that the catheter unit 100 can be used, and permits subsequent operations of the medical device 1 (S103). If the historical information indicates that the catheter unit 100 has not been unused, that is, has been used (NO in S102), the controller 3 determines that the catheter unit 100 cannot be used, and does not permit subsequent operations of the medical device 1 (S104). For example, by using the monitor 4, a message or an image that informs a user that the catheter unit 100 has been used and the medical device 1 cannot be operated may be displayed (S105).

Here, a state in which an operation of the medical device 1 is not permitted is, to be specific, a state in which an operation of the wire driving portion 300 is not permitted and a bending operation of the catheter 11 is suppressed. In this way, it is sufficient that the state in S104 be a state in which a user cannot perform an operation by using the catheter unit 100 that is attached at present. That is, other operations of the medical device 1, such as an operation with which a user checks the status of the medical device 1 by using a button of the input device 3b, the monitor 4, or the like are not hindered.

After the catheter unit 100 has been used, it is necessary to newly write and store, in the information storage 110, historical information indicating that the catheter unit 100 has been used. The controller 3 writes the information in the information storage 110 by using the information communication means 210. Writing of the information may be performed at a timing when it is possible to determine that the catheter unit 100 cannot be used when the catheter unit 100 is reattached after having been detached. For example, the timing may be immediately after the catheter unit 100 is attached to the base unit 200 and the historical information as to whether the catheter unit 100 has not been used or has been used is acquired, or may be a timing when the catheter unit 100 is used and inserted to the inside of a subject. Deletion of historical information indicating that the catheter unit 100 has not been used may be used as historical information indicating that the catheter unit 100 has been used. It is possible to use different addresses in the information storage 110 for historical information indicating that the catheter unit 100 has been used and for historical information indicating that the catheter unit 100 has not been used. In this case, it is not necessary to delete historical information indicating that the catheter unit 100 has not been used.

As described above, in the present embodiment, historical information indicating whether the catheter unit 100 has been used or has not been used is stored in the information storage 110 of the catheter unit 100. Then, whether or not to permit operation of the medical device 1 is determined in such a way that the catheter unit 100 can be used if the information indicates that the catheter unit 100 has not been used and that the catheter unit 100 cannot be used if the information indicates that the catheter unit 100 has been used. By doing so, a catheter unit that has been used is prevented from being mistakenly reused, and thus the medical device can be used safely and hygienically.

### [Second Embodiment]

In the section related to the catheter in the first embodiment, it has been described that the first to third guide rings (J1 to J3) are moved by the first to ninth drive wires (W11 to W33) to bend the bending portion 12. The present invention is not limited to this configuration. One or two of the first to third guide rings (J1 to J3) may be omitted.

For example, the catheter 11 may have a configuration such that: the seventh to ninth drive wires (W31 to W33) and the third guide ring J3 are included; and the first to sixth drive wires (W11 to W23) and first and second guide rings (J1 and J2) are omitted. The catheter 11 may have a configuration such that: the fourth to ninth drive wires (W21 to W33) and the second and third guide rings (J2 and J3) are included; and the first to third drive wires (W11 to W13) and the first guide ring J1 are omitted. This is because, depending on the shape of a path inside of a subject into which the catheter 11 is to be inserted and the purpose of use, guide rings and drive wires W may be omitted as described above to provide an inexpensive catheter unit 100.

The present embodiment is characterized in that, if there exist a plurality of types of catheter units 100 that differ in the number of guide rings, that is, that differ in the region number of bending regions, the controller 3 controls only the drive sources M corresponding to guide rings that exist. Here, the region number of bending regions corresponds to the number of regions where the catheter unit 100 is independently bendable at positions that differ from each other in the longitudinal direction.

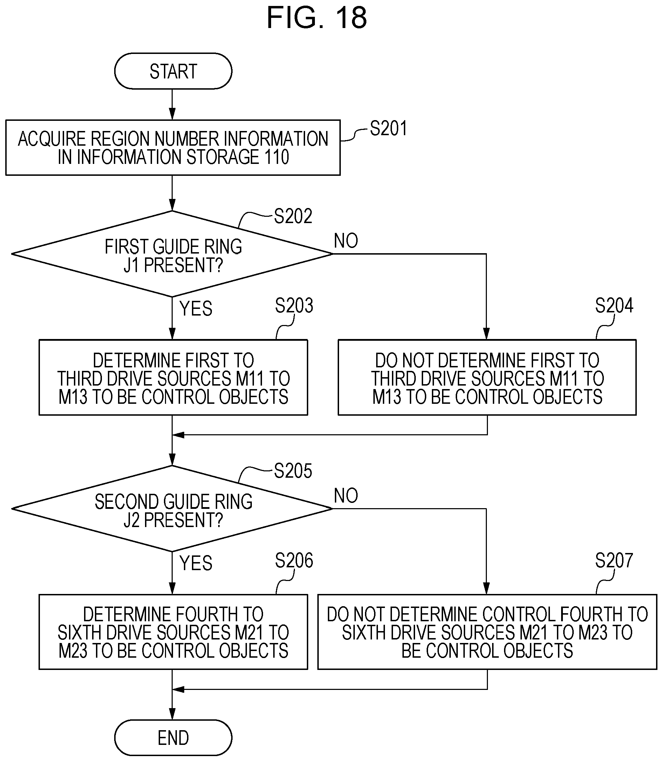

The information storage 110 stores region number information indicating the number of guide rings of the catheter unit 100 (which guide ring among the first to third guide rings is present). Fig. 18 illustrates a flowchart of control according to the present embodiment. The flowchart illustrated in Fig. 18 is realized as a CPU (not shown) included in the controller 3 executes a program stored in a ROM (not shown). The present control corresponds to the start-up check (S003) in the procedure for using the medical system 1A illustrated in Fig. 16.

The controller 3 acquires the region number information stored in the information storage 110 (S201), and determines whether or not the first guide ring J1 is present from the region number information (S202). If the first guide ring J1 is present (YES in S202), since the first to third drive wires (W11 to W13) connected thereto exist, the controller 3 determines the first to third drive sources (M11 to M13), which are coupled thereto, to be control objects (S203). That is, the controller 3 permits operation of the first to third drive sources (M11 to M13) in and after S004 of Fig. 16. If the first guide ring J1 is not present (NO in S202), since the first to third drive wires (W11 to W13) connected thereto do not exist, there is nothing to be coupled to the first to third drive sources (M11 to M13). Thus, since the first to third drive sources (M11 to M13) need not be controlled, the controller 3 does not determine these to be control objects (S204). That is, the controller 3 does not permit operation of the first to third drive sources (M11 to M13) in and after S004 illustrated in Fig. 16.

In this way, according to the present embodiment, if the first guide ring J1 does not exist, the first to third drive sources (M11 to M13) are prevented from being operated unnecessarily. Although the controller 3 usually performs control so as to move the first guide ring J1 to follow the movement of the second guide ring J2 and the third guide ring J3, the controller 3 is prevented from performing such unnecessary operation control. As a result, it is possible to save electric power for causing the first to third drive sources (M11 to M13) to operate, and it is possible to provide the medical system 1A that is configured to save more energy than before.

Next, whether or not the second guide ring J2 is present is determined from the region number information acquired from the information storage 110 (S205). If the second guide ring J2 is present (YES in S205), since the fourth to sixth drive wires (W21 to W23) connected thereto exist, the controller 3 determines the fourth to sixth drive sources (M21 to M23), which are coupled thereto, to be control objects (S206). That is, the controller 3 permits operation of the fourth to sixth drive sources (M21 to M23) in and after S004 of Fig. 16. If the second guide ring J2 is not present (NO in S205), since the fourth to sixth drive wires (W21 to W23) connected thereto do not exist, there is nothing to be coupled to the fourth to sixth drive sources (M21 to M23). Thus, since it is not necessary to control the fourth to sixth drive sources (M21 to M23), the controller 3 does not determine these to be control objects (S207). That is, the controller 3 does not permit operation of the fourth to sixth drive sources (M21 to M23) in and after S004 illustrated in Fig. 16.

In this way, according to the present embodiment, if the second guide ring J2 does not exist, the fourth to sixth drive sources (M21 to M23) are prevented from being operated unnecessarily. Although the controller 3 usually performs control so as to move the second guide ring J2 to follow the movement of the third guide ring J3, the controller 3 is prevented from performing such unnecessary operation control. As a result, it is possible to save electric power for causing the fourth to sixth drive sources (M21 to M23) to operate, and it is possible to provide the medical system 1A that is configured to save more energy than before.

Since the third guide ring J3 is provided at the distal end of the catheter and is necessary for bending control of the catheter, the third guide ring exists in the present embodiment. Thus, the seventh to ninth drive sources (M31 to M33) that drive the third guide ring are necessarily determined to be control objects. This is because at least one bending region exists in the catheter unit 100.

As described above, according to the present embodiment, the controller 3 can determine whether to permit or not to permit operation of each drive source M by using the region number information stored in the information storage 110. That is, it is possible to use the same base unit 200 and the same controller 3 for a plurality of types of catheter units 100 that differ in the number of guide rings. Then, by automatically discriminating it, it is possible to perform optimal control for catheter units 100 of different types.

### [Third Embodiment]

The present embodiment is characterized in that, when there are a plurality of catheter units 100 that differ in the distance between the guide rings, control is performed in accordance with the distance.

As described above, the medical device 1 according to the present invention is supposed to be inserted into a bronchus or the like, and the catheter 11 moves in a long and narrow space. For example, when the catheter 11 is being inserted to the inside of a subject, it is necessary to perform control so that the following bending regions (the second guide ring J2 and the first guide ring J1) pass along a path that is the same as the path of the third guide ring J3, which is the distal end portion of the bending region 12b of the catheter 11. This will be referred to as leader following control, and is realized by causing control of the third guide ring J3, which is the leader, to propagate with a time lag to control of the second guide ring J2 and control of the first guide ring J1 that follow. This time lag is determined by the insertion speed of the catheter 11 and the distance between the guide rings. The distance between the guide rings is the length of the bending region.

The insertion speed of the catheter 11 is the movement speed of the movement stage 2a. On the other hand, the distance between the guide rings is determined by the configuration of the catheter unit 100. In the catheter unit 100, it is necessary that the bend radius with which the bending portion 12 of the catheter 11 is bendable be smaller than the minimum radius of a path into which the catheter 11 is to be inserted. The bend radius of the catheter 11 is determined by the distance between the guide rings, and the bend radius decreases as the distance between the guide rings decreases and increases as the distance increases.

The present embodiment is characterized in that, when there are a plurality of types of catheter units 100 that differ in the distance between the guide rings that are in the bending region 12b of the bending portion 12 of the catheter 11, the controller 3 performs leader following control in accordance with the distance between the guide rings.

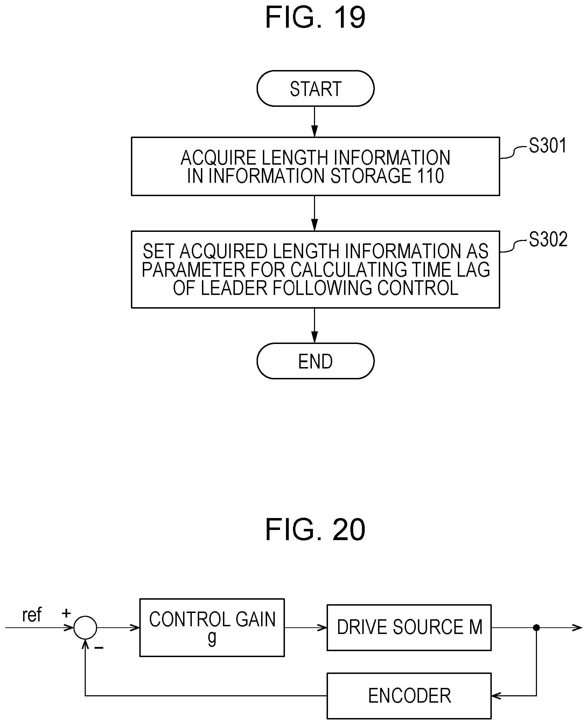

Fig. 19 illustrates a flowchart of control according to the present embodiment. The flowchart illustrated in Fig. 19 is realized as a CPU (not shown) included in the controller 3 executes a program stored in a ROM (not shown). The present control corresponds to the start-up check (S003) in the procedure for using the medical system 1A illustrated in Fig. 16.

The controller 3 acquires length information stored in the information storage 110 (S301). The acquired length information (the distance between the guide rings) is set as a parameter for calculating the time lag of control propagation between the guide rings in leader following control (S302).

For example, when a catheter unit 100A in which the length of a bending region at the distal end portion of the catheter 11 (the distance between the third guide ring J3 and the second guide ring J2), which is to be bent by moving the third guide ring, is comparatively large is attached, control is performed as follows. First, a drive source connected to the third guide ring is operated, and after a predetermined time has elapsed, a drive source connected to the second guide ring is operated.

On the other hand, when a catheter unit 100B in which the length of a bending region at the distal end portion of the catheter 11 (the distance between the third guide ring J3 and the second guide ring J2) is greater than that in the catheter unit 100A is attached, control is performed as follows. First, a drive source connected to the third guide ring is operated, and after a time longer than the predetermined time has elapsed, a drive source connected to the second guide ring is operated.

As described above, according to the present embodiment, the controller 3 can determine the timing for driving each drive source M based on the length information of the bending region stored in the information storage 110. That is, it is possible to use the same base unit 200 and the same controller 3 for a plurality of types of the catheter units 100 that differ in the distance between the guide rings. Then, by automatically discriminating it, it is possible to perform optimal control for catheter units 100 of different types.

### [Fourth Embodiment]

The bending operation of the catheter 11 of the medical device 1 described in the first embodiment is performed by using the nine drive wires W, and the drive sources M that move the drive wires W are controlled independently from each other. When the catheter 11 is to perform a desired bending operation, for example, is to undergo a transition from a certain bending state to a bending state in another orientation, if the operations of the nine drive wires W and the drive sources M are not in synchronism, it is not possible to perform a smooth transition due to generation of unintended vibration or the like.

Each drive wire W extends through the inside of the wire hole Hw in the catheter 11 from the proximal end to the distal end. The nine drive wires W are in different states due to factors such as: a difference in friction amount between the drive wires W and the wire holes Hw due to variation in the diameter, the shape, the rigidity, the surface roughness, and the like of the drive wires W and the wire holes Hw; and twisting of the catheter 11. That is, since the response characteristics of the drive wires W when driven by the drive sources M differ from each other, if optimal control is not performed for each drive wire W, the bending operation of the catheter 11 may be become unstable. The present embodiment is characterized in that information about the response characteristics of each drive wire W is stored in the information storage 110, and the controller 3 performs control of each drive source M based on the response information.

There are various methods for controlling the drive source M, including, for example, a feedback control method illustrated in Fig. 20. The rotation amount of the drive source M that is a control object is detected by an encoder attached to the drive source M, a certain calculation (referred to as a control gain g) is performed on the difference between the detected rotation amount and a target rotation amount, and the driving amount of the drive source M is determined based on this. Thus, the rotation of the drive source M is made to coincide with and follow a target value calculated by the controller 3. Feedback control may be performed by using, as a parameter, a torque generated by the drive source M and a tension generated in the drive wire W, instead of the rotation amount.

The control gain g is determined by measuring the response characteristics of this control system (the catheter unit 100 and the drive source M) beforehand. When the catheter unit 100 is manufactured, response characteristics are measured by attaching the catheter unit 100 to the base unit 200 for measuring response characteristics. The control gain g is calculated and determined from the response characteristics obtained by this measurement, and the value thereof is stored in the information storage 110.

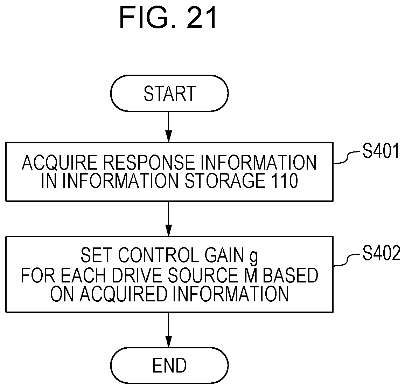

Fig. 21 illustrates a flowchart of control according to the present embodiment. The flowchart illustrated in Fig. 21 is realized as a CPU (not shown) included in the controller 3 executes a program stored in a ROM (not shown). The present control corresponds to the start-up check (S003) in the procedure for using the medical system 1A illustrated in Fig. 16.

The controller 3 acquires the response information stored in the information storage 110 (S401). The controller 3 sets the control gain g for each drive source M based on the response characteristics (the control gain g) of each drive wire W, which is the acquired information (S402). The controller 3 adjusts the driving amount of the drive source M in accordance with the set control gain g in and after S004 of Fig. 16.

As described above, according to the present embodiment, the characteristics of each drive wire W of the catheter unit 100 are independently stored in the information storage 110, and, based on the values thereof, the controller 3 independently sets control parameters of the plurality of drive sources M and performs control. Thus, it is possible to make the characteristics of the drive wires W as uniform as possible and to stably perform a bending operation of the catheter 11.

### [Fifth Embodiment]

As heretofore described, in a medical system according to the present invention, since the catheter unit 100 is replaced every time and used, information specific to the catheter unit 100 is stored in the information storage 110 provided in the catheter unit 100. On the other hand, although the base unit 200 need not be replaced, the base unit 200 may become unusable due to breakage or a malfunction of a component or the like. Therefore, it is effective to configure the base unit 200 and the controller 3 to be attachable/detachable and replaceable in view of reduction of replacement components when a malfunction occurs.

For example, it is possible to realize replacement of the base unit 200 by providing a connector on at least one of the base unit 200 side and the support base 2 side of the cable 5 in Fig. 1 and making the connector detachable. Naturally, another method may be used.

The present embodiment is characterized in that an information storage 220 as a second information storage is provided in the base unit 200, information specific to the base unit 200 is stored, to be specific, information about the output characteristics of the drive sources M in the base unit 200 is stored, and the information is used for control.

In the fourth embodiment described above, variation in the characteristics of the drive wires W is stored in the information storage 110 in the catheter unit 100 and used for control. However, since the feedback control system described above includes the drive sources M, it is necessary to consider variation in the output characteristics of the drive sources M in order to perform further optimal control. However, since the drive sources M are in the base unit 200, information about the output characteristics thereof need to be stored not in the information storage 110 in the catheter unit 100 but in the information storage 220 provided in the base unit 200.

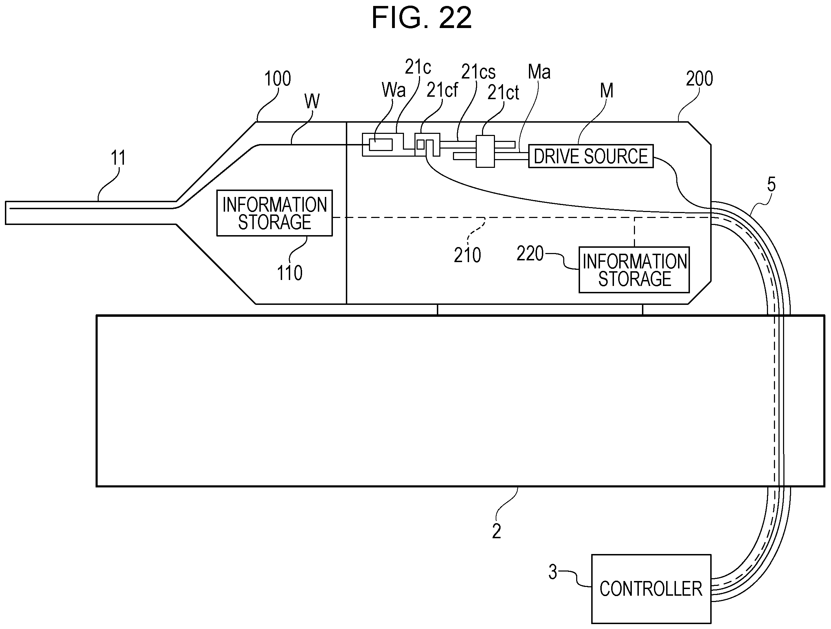

Fig. 22 illustrates a schematic block diagram of the medical device 1 according to the present embodiment. The information storage 220 is provided in the base unit 200. As with the information storage 110 in the catheter unit 100, the information storage 220 is connected to the controller 3 via the information communication means 210. The information communication means 210 may be the same as the information communication means 210 of the information storage 110 in the catheter unit 100 as illustrated in Fig. 22, or may be newly provided independently. The method of communication between the information storage 220 of the base unit 200 and the controller 3 may be either of wired or wireless.

The control gain g of the feedback control system described above includes a portion to which the drive wire W contributes and a portion to which the drive source M contributes. Only the contribution of the drive wire W is considered in the fourth embodiment, and the contribution of the drive source M is considered in the present embodiment. The contribution of the drive source M to the control gain g is calculated from the torque-rotation-speed characteristics and the torque-current characteristics of each drive source M.

Description of the flowchart of control according to the present embodiment, which is the same as that of the fourth embodiment illustrated in Fig. 21 except for the difference between the information storage 110 and the information storage 220, will be omitted. The present control corresponds to the start-up check (S003) in the procedure for using the medical system 1A illustrated in Fig. 16. Since communication between the controller 3 and the information storage 220 is possible before the catheter unit 100 is attached to the base unit 200, the present control may be performed immediately after the medical system 1A illustrated in Fig. 16 is switched on (S002).

As described above, according to the present embodiment, the characteristics of each drive source M in the base unit 200 are independently stored in the information storage 220 provided in the base unit 200, and, based on the values thereof, the controller 3 sets control parameters of the plurality of drive sources M independently and performs control. Thus, it is possible to make the characteristics of the drive sources M as uniform as possible and to stably perform a bending operation of the catheter 11.

### [Sixth Embodiment]

In Fig. 6B of the first embodiment, the force sensor 21cf (detector) that detects a tension generated in the drive wire W is provided in the coupling portion 21c of the base unit 200. The force sensor 21cf is composed of a strain body 21cl and a strain gauge 21csg affixed to the strain body 21cl; and the strain gauge 21csg detects very small elastic deformation of the strain body 21cl that occurs as a tension is generated in the drive wire W, and transmits to the controller 3.

While the catheter 11 is being inserted to the inside of a subject, the catheter 11 may contact or abut against a wall inside of the subject and may press the wall inside of the subject. In such a state, insertion of the catheter 11 may become difficult. Since a tension is generated in each drive wire W at such a time, the tension is detected by using the force sensor 21cf. If a force detected by the force sensor 21cf becomes greater than a predetermined value, control is performed so as to move the drive wire W and bend the catheter 11 so that the value of the force sensor 21cf becomes less than or equal to a certain value, that is, so that the tension is reduced. Other than that, the force sensor 21cf can be used also for a parameter of the feedback control of the drive source M described in the fourth embodiment.

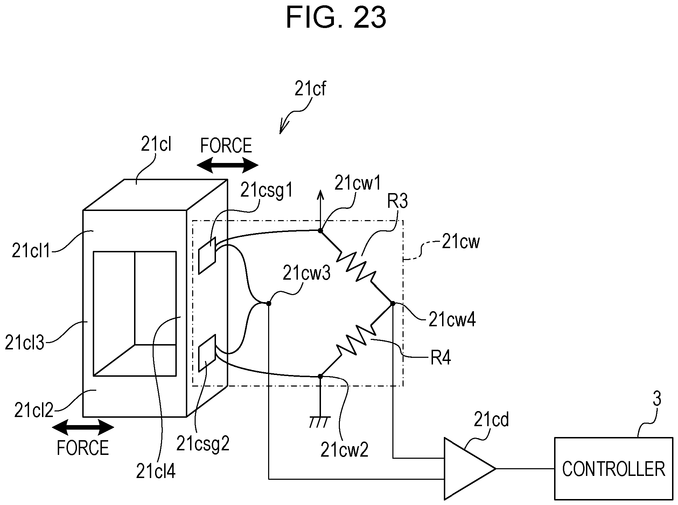

Fig. 23 illustrates a block diagram of the force sensor 21cf. The strain body 21cl is a hollow rectangular metal component having a void at the center thereof. An upper wall 21cl1 and a lower wall 21cl2, which extend in a horizontal direction of Fig. 23, and a left wall 21cl3 and a right wall 21cl4, which extend in the vertical direction of Fig. 23, are provided in the strain body 21cl so as to surround the void.

A tension generated in the drive wire W deforms the strain body 21cl. For example, when the strain body 21cl is pulled outward in the left-right direction, the upper wall 21cl1 moves in the rightward direction, and the lower wall 21cl2 moves in the leftward direction. The left wall 21cl3 and the right wall 21cl4 of the strain body 21cl also elastically deform slightly due to displacement of the upper wall 21cl1 and the lower wall 21cl2. This elastic deformation is detected by the strain gauges 21csg affixed to the left wall 21cl3 and the right wall 21cl4.

In Fig. 23, two strain gauges 21csg1 and 21csg2 are affixed to the right wall 21cl4. The strain gauge 21csg measures the strain amount of an object, to which the strain gauge 21csg is affixed, by deforming in accordance with extension and contraction of the object and changing the resistance value thereof. A Wheatstone bridge circuit 21cw is formed by the strain gauges 21csg1 and 21csg2 and resistors R3 and R4, and a slight change in the resistor values of the strain gauges 21csg1 and 21csg2 is converted into a voltage. A voltage is applied between input terminals 21cw1 and 21cw2 of the Wheatstone bridge circuit 21cw, a potential difference generated between output terminals 21cw3 and 21cw4 is amplified by using a differential amplifier 21cd, and the output voltage thereof is output to the controller 3. The controller 3 calculates the value of a tension generated in the drive wire W based on this detection value.

Although two strain gauges 21csg are affixed in the present embodiment, strain of the strain body 21cl can be detected as long as one or more of the four resistors forming the Wheatstone bridge circuit 21cw is a strain gauge. Therefore, it is sufficient that the number of the strain gauge 21csg be one or more. Although the strain gauges 21csg are affixed to only the right wall 21cl4 in Fig. 23, a strain gauge 21csg may be affixed also to the left wall 21cl3. Two strain gauges 21csg may be affixed also to the left wall 21cl3, and the remaining resistors R3 and R4 of the Wheatstone bridge circuit 21cw may be substituted with the strain gauges 21csg on the left wall 21cl3.

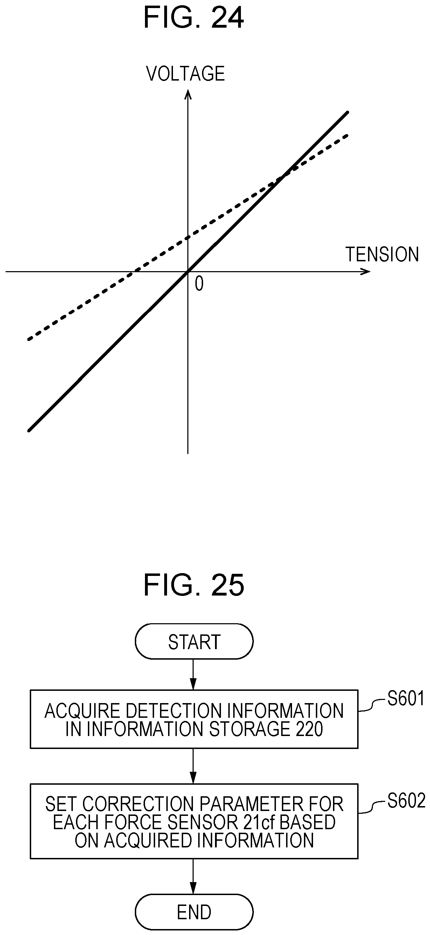

As an output of the force sensor 21cf, a voltage proportional to a tension is generated. Fig. 24 is a graph illustrating detection characteristics, with the tension on the horizontal axis and the output voltage of the force sensor 21cf on the vertical axis. As shown by the solid line in the figure, ideally, the output voltage is zero when the tension is zero, and the graph has a predetermined slope. However, as shown by the dotted line in the figure, the characteristics become offset and the slope changes due to variation in the resistor values of the strain gauges 21csg and the resistors R3 and R4 and the accuracy (in position and inclination) of affixing of the strain gauges 21csg to the strain body 21cl. Since the variation in characteristics affects the accuracy of tension detection, the present embodiment is characterized in that the variation is stored as detection information in the information storage 220 of the base unit 200 in which the force sensor 21cf is disposed. Measurement of the characteristics of the force sensor 21cf is performed, when the force sensor 21cf is being manufactured or when the base unit 200 is being manufactured, by using a measurement tool that is produced to be capable of applying a force to the force sensor 21cf.

Fig. 25 illustrates a flowchart of control according to the present embodiment. The flowchart illustrated in Fig. 25 is realized as a CPU (not shown) included in the controller 3 executes a program stored in a ROM (not shown). The present control corresponds to the start-up check (S003) in the procedure for using the medical system 1A illustrated in Fig. 16. Since communication between the controller 3 and the information storage 220 is possible before the catheter unit 100 is attached to the base unit 200, the present control may be performed immediately after the medical system 1A is switched on (S002).

The controller 3 acquires the detection information stored in the information storage 220 (S601). Based on the detection characteristics (offset amount and slope amount) of each force sensor 21cf, which are the acquired detection information, a parameter for correcting the detection value (detection result) of each force sensor 21cf is set (S602).

As described above, according to the present embodiment, the characteristics of each force sensors 21cf in the base unit 200 are independently stored in the information storage 220 provided in the base unit 200, and, based on the values thereof, the controller 3 automatically corrects the detection value of each force sensor 21cf. Thus, it is possible to increase the detection accuracy of the force sensor 21cf as much as possible.

### [Seventh Embodiment]

If a component in the base unit 200, such as the drive source M or the force sensor 21cf, malfunctions, the controller 3 needs to make the base unit 200 unusable. The present embodiment is characterized in that, if a malfunction of a component in the base unit 200 occurs, malfunction information is stored in the information storage 220 in the base unit 200, and the controller 3 does not permit operation of the medical device 1 when acquiring the information.

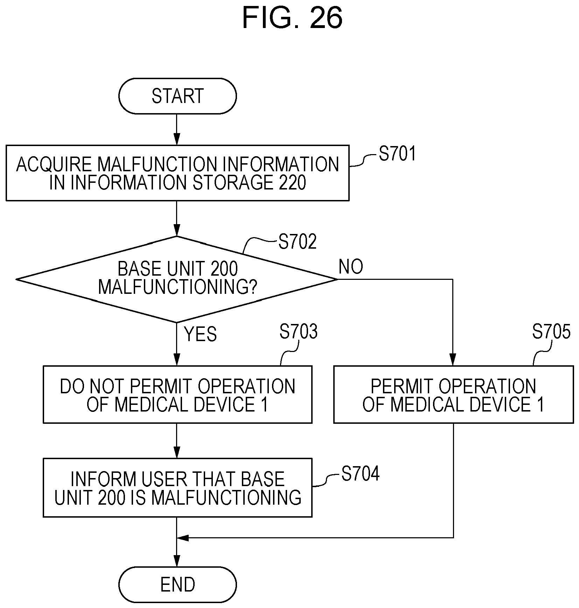

Fig. 26 illustrates a flowchart of control according to the present embodiment. The flowchart illustrated in Fig. 26 is realized as a CPU (not shown) included in the controller 3 executes a program stored in a ROM (not shown). The present control corresponds to the start-up check (S003) in the procedure for using the medical system 1A illustrated in Fig. 16. Since communication between the controller 3 and the information storage 220 is possible before the catheter unit 100 is attached to the base unit 200, the present control may be performed immediately after the medical system 1A is switched on (S002).

The controller 3 acquires the malfunction information stored in the information storage 220 (S701). If the acquired malfunction information indicates that a malfunction has occurred (YES in S702), the controller 3 does not permit operation of the medical device 1 (S703), and informs a user that the base unit 200 is malfunctioning and unusable (S704). At this time, the controller 3 may use the monitor 4 to display a message or an image that informs a user that the base unit 200 is malfunctioning and the medical device 1 cannot be operated. If the acquired malfunction information does not indicate that a malfunction has occurred (NO in S702), the controller 3 permits operation of the medical device 1 since it is possible to use the medical device 1 (S705).

As described above, the present control is performed in the start-up check of the medical system 1A (S003), but, if it is detected that a malfunction has occurred while the medical system 1A is being operated, the operation of the medical device 1 is immediately stopped. Then, the controller 3 writes and stores malfunction occurrence information in the information storage 220 in the base unit 200. After the malfunction has been resolved, for example, an authorized person such as a service person deletes the malfunction occurrence information in the information storage 220.

As described above, according to the present embodiment, the malfunction occurrence information about a malfunction that has occurred in the base unit 200 is stored in the information storage 220, and the controller 3 stops an operation when acquiring the malfunction information and makes unusable. Thus, it is possible to prevent a user from causing an unintended trouble by using the medical device 1 that is malfunctioning.

In the first to fourth embodiments described above, the controller 3 performs different controls based on information specific to the catheter unit 100 stored in the information storage 110. Here, two or more of the controls in the first to fourth embodiments may be combined and performed.

In the fifth to seventh embodiments, the controller 3 performs different controls based on information specific to the base unit 200 stored in the information storage 220. Here, two or more of the controls in the fifth to seventh embodiments may be combined and performed.

Two or more of the controls based on information specific to the catheter unit 100 in the first to fourth embodiments and the controls based on information specific to the base unit 200 in the fifth to seventh embodiments may be combined and performed.

The present invention is not limited to the embodiments described above, and can be changed and modified in various ways without departing from the spirit and scope of the present invention. Accordingly, the following claims are attached to disclose the scope of the present invention.

This application claims priority based on Japanese Patent Application No. 2022-033258 filed March 4, 2022, which is hereby incorporated by reference herein in its entirety.

## Claims

1. A medical system comprising:
a base unit including a drive portion therein;
a bendable unit that is attachable to and detachable from the base unit, the bendable unit including a linear member that is driven by the drive portion in a state in which the bendable unit is attached to the base unit and a bending portion that bends as the linear member is driven; and
a controller that controls the drive portion,
wherein the bendable unit includes a first information storage that stores first information specific to the bendable unit,
wherein the first information includes historical information indicating whether the bendable unit has not been used or has been used, and
wherein, if the historical information indicates that the bendable unit has not been used, the controller permits operation of the drive portion, and, if the historical information indicates that the bendable unit has been used, the controller does not permit operation of the drive portion.

2. A medical system comprising:
a base unit including a drive portion therein;
a bendable unit that is attachable to and detachable from the base unit, the bendable unit including a linear member that is driven by the drive portion in a state in which the bendable unit is attached to the base unit and a bending portion that bends as the linear member is driven; and
a controller that controls the drive portion,
wherein the bendable unit includes a first information storage that stores first information specific to the bendable unit,
wherein the first information includes region number information indicating the number of regions in which the bending portion is independently bendable at different positions in a longitudinal direction of the linear member,
wherein the drive portion includes a first drive source for driving a first bending region and a second drive source for driving a second bending region that is closer than the first bending region to the base unit in the longitudinal direction, and
wherein, if the region number information indicates a first region number, the controller permits operation of the first drive source and does not permit operation of the second drive source, and, if the region number information indicates a second region number that is greater than the first region number, the controller permits operation of the first drive source and the second drive source.

3. A medical system comprising:
a base unit including a drive portion therein;
a bendable unit that is attachable to and detachable from the base unit, the bendable unit including a linear member that is driven by the drive portion in a state in which the bendable unit is attached to the base unit and a bending portion that bends as the linear member is driven; and
a controller that controls the drive portion,
wherein the bendable unit includes a first information storage that stores first information specific to the bendable unit,
wherein the first information includes length information indicating lengths of regions in which the bending portion is independently bendable at different positions in a longitudinal direction of the linear member,
wherein the drive portion includes a first drive source for driving a first bending region and a second drive source for driving a second bending region that is closer than the first bending region to the base unit in the longitudinal direction, and
wherein, if the length information indicates that a length of the first bending region is a first length, the controller causes the second drive source to operate at a timing when a predetermined time has elapsed after driving the first drive source, and, if the length information indicates that the length of the first bending region is a second length that is greater than the first length, the controller causes the second drive source to operate at a timing when a time longer than the predetermined time has elapsed after driving the first drive source.

4. A medical system comprising:
a base unit including a drive portion therein;
a bendable unit that is attachable to and detachable from the base unit, the bendable unit including a linear member that is driven by the drive portion in a state in which the bendable unit is attached to the base unit and a bending portion that bends as the linear member is driven; and
a controller that controls the drive portion,
wherein the bendable unit includes a first information storage that stores first information specific to the bendable unit,
wherein the first information includes information about response characteristics when the drive portion drives the linear member, and
wherein the controller adjusts a driving amount of the drive portion based on the information about the response characteristics.

5. The medical system according to any one of Claims 1 to 4,
wherein, in a medical system in which the base unit and the controller are attachable to and detachable from each other,
the base unit includes a second information storage that is different from the first information storage, and the second information storage stores second information specific to the base unit.

6. The medical system according to Claim 5,
wherein the second information includes information about output characteristics of the drive portion, and
wherein the controller adjusts a driving amount of the drive portion based on the information about the output characteristics.

7. The medical system according to Claim 5 or 6,
wherein the base unit includes a detector that detects a tension generated in the linear member,
wherein the second information includes information about detection characteristics of the detector, and
wherein the controller corrects a detection result by the detector based on the information about the detection characteristics.

8. The medical system according to any one of Claims 5 to 7,
wherein the second information includes malfunction information indicating whether or not a malfunction has occurred in a component included in the base unit, and
wherein, if the malfunction information indicates that a malfunction has occurred in a component included in the base unit,
the controller does not permit operation of the drive portion, and, if the malfunction information indicates that a malfunction has not occurred in a component included in the base unit, the controller permits operation of the drive portion.
